# EUROPEAN PATENT APPLICATION

(11) **EP 4 206 328 A1**
(43) Date of publication of application: **05.07.2023**
(21) Application number: 21860450.2
(22) Date of filing: 25.08.2021
(51) Int. Cl.: C12P 19/30

(54) **YEAST POWDER RICH IN NICOTINAMIDE MONONUCLEOTIDE, PREPARATION METHOD THEREFOR, AND APPLICATION THEREOF**

(30) Priority: 25.08.2020 US 202063069729 P
(71) Applicant: TCI Co., Ltd, Taipei 11494 (CN)
(72) Inventor: LIN, Yung-hsiang, 11494 Taipei Taiwan (CN); WU, Pei-yi, 11494 Taipei Taiwan (CN)
(74) Representative: Viering, Jentschura & Partner mbB Patent- und Rechtsanwälte
(86) International application number: PCT/CN2021/114593
(87) International publication number: WO 2022/042615

(57) **Abstract**

The present invention discloses a yeast powder rich in nicotinamide mononucleotide (NMN), and a preparation method and applications thereof. The preparation method includes: preparing a first medium, a second medium and a third medium; inoculating the first medium with yeast for fermentation to obtain a first fermentation broth; inoculating the second medium with the first fermentation broth for fermentation to obtain a second fermentation broth; inoculating the third medium with the second fermentation broth for fermentation to obtain a third fermentation broth; and centrifuging the third fermentation broth to obtain a fermented product, and drying the fermented product to obtain the yeast powder. Components of the first medium, the second medium and the third medium include nicotinamide (NAM), tryptophan and niacin. The content of NMN in the yeast powder is at least 5000 ppm.

## Description

### BACKGROUND

### Technical Field

The present invention relates to a yeast powder, in particular to a preparation method of the yeast powder and the yeast powder prepared by the preparation method. The yeast powder is rich in NMN and has multiple applications.

### Related Art

Studies have found that nicotinamide adenine dinucleotide (NAD⁺) is related to anti-cell aging, and the NAD⁺ has the main physiological functions of generating more than 95% life activity energy, repairing damaged DNA, activating long-lived protein, etc., and serves as a component for maintaining the activity of mitochondria *in vivo.*

However, NAD⁺ cannot be obtained directly from the diet, and as humans age, the amount of NAD⁺ naturally generated *in vivo* is reduced. Therefore, in order to supplement and maintain the content of NAD⁺ *in vivo,* studies have found that precursors of NAD⁺ can be ingested to increase the content of NAD⁺ *in vivo.*

For example, after NMN is ingested, NMN can be converted into NAD⁺ *in vivo* through blood to accelerate the removal of aging substances accumulated *in vivo.*

### SUMMARY

Since the content of natural NMN in plants (such as broccoli and avocado) is very low and the natural NMN is not easy to extract, most of commercially available NMN is a chemically synthesized formula at present. However, the process of chemically synthesizing NMN is accompanied by some by-products, and these by-products exist in NMN products. Moreover, when such NMN products are ingested by the human body, these by-products are easy to cause metabolic burden of the human liver.

In view of this, the present invention provides a preparation method of a yeast powder rich in NMN and the yeast powder prepared by the preparation method, and provides applications of the yeast powder prepared by the preparation method for improvement of skin conditions, hair care, anti-inflammation, cardiovascular health care, antioxidation, antiaging, and/or improvement of body fatigue.

In some embodiments, provided is a preparation method of a yeast powder rich in NMN, including: preparing a first medium, a second medium and a third medium; inoculating the first medium with yeast for fermentation to obtain a first fermentation broth; inoculating the second medium with the first fermentation broth for fermentation to obtain a second fermentation broth; taking the second fermentation liquor, and inoculating the third medium with the second fermentation broth for fermentation to obtain a third fermentation broth; and centrifuging the third fermentation broth to obtain a fermented product, and drying the fermented product to obtain the yeast powder. Components of the first medium, the second medium and the third medium include nicotinamide (NAM), tryptophan and niacin. The content of NMN in the yeast powder is at least 5000 ppm.

In some embodiments, the first medium is inoculated with the yeast at an inoculation amount of 5 vol%, the second medium is inoculated with the first fermentation broth at an inoculum amount of 6 vol%, and the third medium is inoculated with the second fermentation broth at an inoculum amount of 10 vol%.

In some embodiments, the concentration of the NAM is 0.01-0.3 wt%, the concentration of the tryptophan is 0.1-0.5 wt%, and the concentration of the niacin is 0.01-0.06 wt%.

In some embodiments, the concentration of the NAM is 0.1 wt%, the concentration of the tryptophan is 0.2 wt%, and the concentration of the niacin is 0.0369 wt%.

In some embodiments, the volume ratio of the first medium to the second medium to the third medium is 3:50:500.

In some embodiments, components of the first medium, the second medium and the third medium further include any one of a *Musa spp.* peel extract, an *Aronia melanocarpa* extract and a *Solanum lycopersicum* 'Kumato' extract.

In some embodiments, provided is a yeast powder prepared by the preparation method according to claim 1, and the content of NMN in the yeast powder is at least 5000 ppm.

In some embodiments, provided is an application of a yeast powder for preparing a composition for improvement of skin conditions, hair care, anti-inflammation, cardiovascular health care, antioxidation, antiaging, and/or improvement of body fatigue, where the yeast powder is prepared by the preparation method according to claim 1, and the content of NMN in the yeast powder is at least 5000 ppm.

In some embodiments, the improvement of skin conditions is to enhance skin tightness, reduce wrinkles, reduce skin roughness, or combinations thereof.

In some embodiments, the hair care is to reduce hair loss or reduce the degree of hair scantiness.

In some embodiments, the yeast powder further includes Q₁₀ biosynthetic protein (COQ protein).

In some embodiments, the yeast powder is used to regulate blood fat.

In some embodiments, the yeast powder is used to reduce the expression of C-reactive protein (CRP) of a subject.

In some embodiments, the yeast powder achieves the antiaging function by regulating the expression of anti-aging related genes, activating mitochondria or reducing brain age.

In some embodiments, the anti-aging related genes include *CCT* genes, *PARP2* gene, *Parkin* gene, *Atg* genes, *FOXO* gene, *SIRT1* gene, *NADSYN* gene, *MRPS5* gene, *SOD3* gene or combinations thereof.

In some embodiments, the yeast powder achieves the antioxidation function by reducing the damage to reactive oxygen species (ROS).

In some embodiments, the daily dose of the yeast powder is 100 mg.

In conclusion, the yeast powder rich in NMN can be prepared according to the preparation method of the yeast powder rich in NMN in any embodiment, and the content of NMN in the yeast powder is at least 5000 ppm. The preparation method solves the problem that the traditional NMN can only be chemically synthesized and thus harmful by-products are generated, and also solves the technical bottleneck that the traditional NMN is inedible and can only be used externally. Moreover, the yeast powder prepared by the preparation method of the yeast powder rich in NMN in any embodiment has the functions of improvement of skin conditions (such as enhancing skin tightness, reducing wrinkles and reducing skin roughness), hair care(such as reducing hair loss and reducing degree of hair scantiness), anti-inflammation, cardiovascular health care, antioxidation, antiaging, and/or improvement of body fatigue, and can be used for preparing a composition with the above functions. In some embodiments, the yeast powder further includes Q₁₀ biosynthetic protein (COQ protein). In some embodiments, the yeast powder has one or any combination of the functions of regulating blood fat, reducing the expression level of CRP of a subject, regulating the expression of anti-aging related genes (such as *CCT*genes, *PARP2* gene, *Parkin* gene, *Atg* genes, *FOXO* gene, *SIRT1* gene, *NADSYN* gene, *MRPS5* gene, *SOD3* gene or combinations thereof), activating mitochondria or reducing brain age to resist aging, and achieving antioxidation by reducing the damage of ROS.

The present invention is described in detail below with reference to the drawings and specific embodiments which are not used as a limitation of the present invention.

### BRIEF DESCRIPTION OF THE DRAWINGS

FIG. 1 is a flow diagram of preparation of a yeast powder rich in NMN.
FIG. 2 is a diagram of experimental results of different strains for yield analysis of NMN.
FIG. 3 is a diagram of experimental results of different fermentation medium components for yield analysis of NMN.
FIG. 4 is a diagram of experimental results of plant extracts and compounds for yield analysis of NMN.
FIG. 5 is a diagram of experimental results of output relative to ROS.
FIG. 6 is a diagram of experimental results of a test for activity of mitochondria.
FIG. 7 is a diagram of experimental results of the relative expression of *PARP2* gene.
FIG. 8 is a diagram of experimental results of the relative expression of *CCT* related genes.
FIG. 9 is a diagram of experimental results of the relative expression of anti-aging related genes.
FIG. 10 is a diagram of experimental results of the relative expression of *NADSYN* gene and *MRPS5* gene.
FIG. 11 is a diagram of experimental results of the relative expression of anti-aging related genes at week 0 and week 8.
FIG. 12 is a diagram of experimental results of the content of CRP at week 0 and week 8.
FIG. 13 is a diagram of experimental results of the content of LDL-C at week 0 and week 8.
FIG. 14 is a diagram of experimental results of the ratio of LDL-C to HDL-C at week 0 and week 8.
FIG. 15 is a diagram of experimental results of the percentage of wrinkles at week 0 and week 8.
FIG. 16 is a diagram of experimental results of the percentage of skin textures at week 0 and week 8.
FIG. 17 is a diagram of experimental results of the brain age at week 0 and week 8.
FIG. 18 is a diagram of experimental results of comprehensive evaluation of physical conditions at week 0 and week 8.

### DETAILED DESCRIPTION

In the description of the following embodiments, unless otherwise specified, the symbol "%" refers to the weight percentage, and the symbol "vol%" usually refers to the volume percentage concentration. In addition, the following serial terms such as "first", "second" and "third" are used for distinguishing the involved components, are not used for sorting or limiting the differences between the involved components, and are not used for limiting the scope of the present invention.

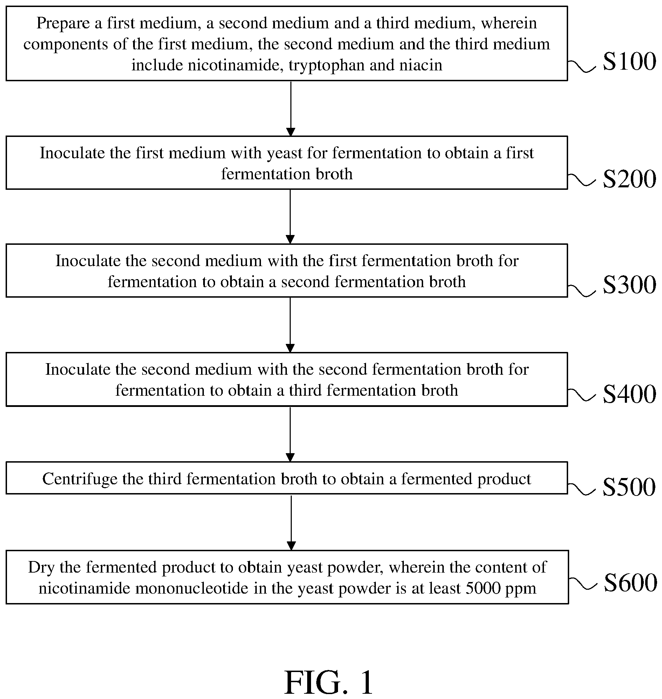

Referring to FIG. 1, first, a first medium, a second medium and a third medium are prepared (step S100). Herein, the components of the first medium, the second medium and the third medium are the same, and include a basal medium, NAM, tryptophan, niacin, etc. For example, the first medium, the second medium and the third medium are fermentation media for fermentation, and the components of the first medium, the second medium and the third medium include a basal medium composed of yeast peptone, yeast extract, potassium dihydrogen phosphate (KH₂PO₄), potassium hydrogen phosphate (KH₂PO₄), magnesium sulphate-7hydrate (MgSO₄·7H₂O), glucose, citric acid, sodium acetate, manganese gluconate, cysteine, deionized distilled water (ddH₂O) and defoamer, and NAM, tryptophan, niacin, etc. In some embodiments, glucose is one of the components of the basal medium, but the glucose is sterilized separately from other components before a fermentation medium is inoculated with a strain, and mixed with other components before the strain is inoculated to form a sterilized fermentation medium.

In some embodiments, the components of the first medium, the second medium and the third medium include 0.01-0.3% of NAM, 0.1-0.5% of tryptophan and 0.001-0.06% of niacin in addition to the basal medium. Preferably, the components of the first medium, the second medium and the third medium include 0.1% of NAM, 0.2% of tryptophan and 0.0369% of niacin in addition to the basal medium. Therefore, when the fermentation medium contains NAM, tryptophan and niacin as precursors for synthesis of NMN, it is beneficial for yeasts to synthesize NMN.

In some embodiments, the components of the first medium, the second medium and the third medium further include any one or more of *Musa* plant extracts, *Aronia* plant extracts and *Solanum* plant extracts. Herein, the *Musa* plant extract may be a *Musa spp.* peel extract, the *Aronia* plant extract may be an *Aronia melanocarpa* extract, and the *Solanum* plant extract may be a *Solanum lycopersicum* 'Kumato' extract. For example, in addition to the basal medium, NAM, tryptophan and niacin, when 0.05% of *Musa spp.* peel extract, 0.085% of *Aronia melanocarpa* extract or 0.05% of *Solanum lycopersicum* 'Kumato' extract is additionally added to the components of the fermentation medium, it is beneficial to increase the content of NMN in the fermentation broth.

In some embodiments, the *Musa spp.* peel extract can be provided by separating peel from pulp of *Musa spp.* and performing the following operations on *Musa spp.* peel: 1. the *Musa spp.* peel is mixed with an extraction solvent (the volume ratio of the *Musa spp.* peel to the extraction solvent is 1:6, where the extraction solvent is provided in the volume ratio of citric acid:water=1:100), and extraction is performed at 85°C for 0.5 hours (h) to provide a crude extract; 2. the crude extract in step 1 is taken and centrifuged at the rotating speed of 5000 rpm for 10 min, and a supernatant is taken and then filtered with a 400-mesh filter screen to provide a filtrate; 3. at 55±5°C, the filtrate in step 2 is concentrated under reduced pressure to provide a concentrated extract; and 4. the concentrated extract in step 3 is freeze-dried to provide a dried product (that is, the above *Musa spp.* peel extract). In some embodiments, the *Aronia melanocarpa* extract is obtained by juicing the fruits of *Aronia melanocarpa* (English name: Black chokeberry) and concentrating. In some embodiments, the *Solanum lycopersicum* 'Kumato' extract is obtained by cutting Solanum lycopersicum 'Kumato' into pieces and homogenizing and grinding the pieces to form a *Solanum lycopersicum* 'Kumato' homogenate, and then extracting the *Solanum lycopersicum* 'Kumato' homogenate with water at 40-60°C for 0.5-2 h, where the liquid-solid ratio of the water to the *Solanum lycopersicum* 'Kumato' homogenate is (5-20):(1-5).

In some embodiments, the volume ratio of the first medium to the second medium to the third medium is 3:50:500.

Step S100 is continued. The first medium is inoculated with yeast for fermentation to obtain a first fermentation broth (step S200). Herein, the yeast used may be *Saccharomyces cerevisiae.* For example, the *Saccharomyces cerevisiae* may be commercially available *Saccharomyces cerevisiae* or *Saccharomyces cerevisiae* TCI907, where the *Saccharomyces cerevisiae* TCI907 is a strain of *Saccharomyces cerevisiae* separated from draft beer. The *Saccharomyces cerevisiae* TCI907 is deposited in the Food Industry Research and Development Institute (FIRDI) with the Deposit No. BCRC 920118, and deposited in the German Collection of Microorganisms and Cell Cultures with the Deposit No. DSZ33480. The *Saccharomyces cerevisiae* TCI907 is an aerobic yeast which is oval. The colony of the *Saccharomyces cerevisiae* TCI907 is opaque milky white, and the colony has a smooth surface and neat edges. The growth temperature of the *Saccharomyces cerevisiae* TCI907 is 28-37°C. Moreover, the *Saccharomyces cerevisiae* TCI907 can survive in an environment with a pH value of 3 to 7. In some embodiments, the *Saccharomyces cerevisiae* TCI907 has the function of resisting gastric acid and bile salt. For example, the survival rate of the *Saccharomyces cerevisiae* TCI907 in a gastric simulated environment (with a pH value of 3 to 4) is 99.4%, and the survival rate of the *Saccharomyces cerevisiae* TCI907 in an intestinal simulated environment (with a pH value of 7) is 99.8%.

In some embodiments, the first medium is inoculated with yeast at an inoculum amount of 5 vol%. For example, before inoculating with the yeast, 3 L of the first medium is prepared and sterilized at the high temperature of 121°C for 30 min. Moreover, after determining that the components contained in the first medium are completely dissolved and the temperature is reduced, 5 vol% (equivalent to 150 mL) of the yeast is inoculated into the first medium for subsequent fermentation. Herein, the OD₆₀₀ of the yeast used for inoculation is 6-8.

In some embodiments, during the fermentation of the first fermentation broth, the fermentation temperature is 30±1°C, the pH value is 6.5±0.1, and the dissolved oxygen (DO) value is maintained at 40-50 mg/L. Moreover, the pH value of the first fermentation broth is regulated by 10 N sodium hydroxide (NaOH).

Moreover, the first fermentation broth is fermented in a fermentation tank. For example, 3 L of the first medium and 150 ml of the yeast may be fermented in a 5 L fermentation tank. In some embodiments, during the fermentation of the first fermentation broth, the ventilation volume is set as 3 L/min, and the stirring rate is set as 250 rpm, thereby ensuring that the fermentation process is performed in an aerobic environment. In addition, before starting the fermentation, the first medium must be stirred at an aeration rate of 3 L/min and a stirring rate of 250 rpm to make the dissolved oxygen greater than 40% before inoculation.

In some embodiments, the fermentation time required for the first fermentation broth is 4-5 h, and the OD₆₀₀ of the yeast in the first fermentation broth after fermentation is 4.0-5.0.

Step S200 is continued. The second medium is inoculated with the first fermentation broth for fermentation to obtain a second fermentation broth (step S300).

In some embodiments, the second medium is inoculated with the first fermentation broth at an inoculum amount of 6 vol%. For example, before inoculating with the first fermentation broth, 50 L of the second medium is prepared and sterilized at the high temperature of 121°C for 25-30 min. Moreover, after determining that the components contained in the second medium are completely dissolved and the temperature is reduced, 6 vol% (equivalent to 3 L) of the first fermentation broth is inoculated into the second medium for subsequent fermentation.

In some embodiments, during the fermentation of the second fermentation broth, the fermentation temperature is 30±1°C, the pH value is 6.5±0.1, and the DO value is maintained at 40-50 mg/L. Moreover, the pH value of the second fermentation broth is regulated by 10 N NaOH.

Moreover, the second fermentation broth is fermented in a fermentation tank. For example, 50 L of the second medium and 3 L of the first fermentation broth may be fermented in a 75 L fermentation tank. In some embodiments, during the fermentation of the second fermentation broth, the ventilation volume is set as 50 L/min, the stirring rate is set as 200 rpm, and the pressure value of the tank pressure in the fermentation tank is 0.3±0.1 kg/cm², thereby ensuring that the fermentation process is performed in an aerobic environment. In addition, before starting the fermentation, the second medium must be stirred at an aeration rate of 30 L/min and a stirring rate of 200 rpm to make the dissolved oxygen greater than 40% before inoculation.

In some embodiments, the fermentation time required for the second fermentation broth is 4-5 h, and the OD₆₀₀ of the yeast in the second fermentation broth after fermentation is 4.0-5.0.

Step S300 is continued. The third medium is inoculated with the second fermentation broth for fermentation to obtain a third fermentation broth (step S400).

In some embodiments, the third medium is inoculated with the second fermentation broth at an inoculum amount of 10 vol%. For example, before inoculating with the second fermentation broth, 500 L of the second medium is prepared and sterilized at the high temperature of 121°C for 30 min. Moreover, after determining that the components contained in the third medium are completely dissolved and the temperature is reduced, 10 vol% (equivalent to 50 L) of the second fermentation broth is inoculated into the third medium for subsequent fermentation.

In some embodiments, during the fermentation of the third fermentation broth, the fermentation temperature is 30±1°C, the pH value is 6.5±0.1, and the DO value is maintained at 40-60 mg/L. Moreover, the pH value of the third fermentation broth is regulated by 10 N NaOH.

Moreover, the third fermentation broth is fermented in a fermentation tank. For example, 500 L of the third medium and 50 L of the second fermentation broth may be fermented in a 750 L fermentation tank. In some embodiments, during the fermentation of the third fermentation broth, the ventilation volume is set as 500 L/min, the stirring rate is set as 100 rpm, and the pressure value of the tank pressure in the fermentation tank is 0.3±0.1 kg/cm², thereby ensuring that the fermentation process is performed in an aerobic environment. In addition, before starting the fermentation, the third medium needs to be stirred at the ventilation volume of 100 L/min and the stirring rate of 200 rpm, and after the DO of the third medium is greater than 40%, the strain can be inoculated.

In some embodiments, the fermentation time required for the third fermentation broth is 12-14 h, and the OD₆₀₀ of the yeast in the third fermentation broth after fermentation is 48.0-53.0.

Step S400 is continued. The third fermentation broth is centrifuged to obtain a fermented product (step S500). In some embodiments, a fermented product is separated from the third fermentation broth by a centrifuge, the OD₆₀₀ of the fermented product is greater than 500, and the solid content of the fermented product is greater than 65% and less than 80%.

In some embodiments, the pH value during centrifugation is 6.5±0.1. In some embodiments, the tank pressure of a centrifuge with 750 L volume is 1.0 kg/cm², and the set flow is 0.3 m³/h.

Herein, the "fermented product" refers to a fermented product of the yeast, including single cell protein of the yeast, protein of the yeast (from a small number of yeasts broken in the process) and a fermentation broth containing metabolites of the yeast (including a medium), where the metabolites of the yeast refer to substances secreted by the yeast to the fermentation medium during culture, such as NMN, coenzyme Q₁₀ and other multiple compounds. For example, the fermented product contains NMN, Q₁₀ biosynthetic protein (COQ protein), coenzyme Q₁₀ and other multiple compounds.

Step S500 is continued. The fermented product is dried to obtain the yeast powder (step S600). Herein, the obtained yeast powder at least contains 1.0×10⁹ CFU/g of the yeast. For example, drying methods include freeze-drying, low-temperature drying, dehydration drying, etc.

In some embodiments, a crude fermented product is freeze-dried and pulverized to obtain a fermented product. Moreover, during the freeze-drying step, a freeze-drying protective agent will be added to protect the single cell protein in the crude fermented product from being damaged. For example, the freeze-drying protective agent includes skim milk powder, maltodextrin, sucrose and glycerol.

Herein, the content of NMN in the obtained yeast powder is at least 5000 ppm, preferably 5000-10000 ppm. For example, the content of NMN in the yeast powder is 5264.15 ppm.

In some embodiments, the yeast powder further includes Q₁₀ biosynthetic protein (COQ protein). The Q₁₀ biosynthetic protein can help the yeast to synthesize Q₁₀ protein, so when the content of the Q₁₀ biosynthetic protein is increased, the content of the coenzyme Q₁₀ synthesized in the yeast can also be increased. Therefore, the yeast powder may further contain the coenzyme Q₁₀.

In some embodiments, compared with the yeast powder prepared by fermentation media without NAM, tryptophan and niacin, the yeast powder prepared by the fermentation media (that is, the first medium, the second medium and the third medium) with components such as NAM, tryptophan and niacin according to the above preparation process can increase the content of NMN at least twice.

In some embodiments, the content of NAM in the fermentation media (that is, the first medium, the second medium and the third medium) is 0.1%, and the yeast has a better yield of NMN. Moreover, when the content of NAM in the fermentation medium is greater than 1.1%, the yield of NMN produced by the yeast is reduced to or lower than the yield of NMN in the medium without adding NAM.

In some embodiments, when the fermentation medium is added with any one or more of *Solanum* plant extracts (such as a *Solanum lycopersicum* 'Kumato' extract), *Aronia* plant extracts (such as an *Aronia melanocarpa* extract) and *Musa* plant extracts (such as a *Musa spp.* peel extract), it is beneficial to increase the content of NMN in the prepared yeast powder. For example, the yeast powder prepared by the fermentation medium which further contains the *Musa spp.* peel extract can increase the content of NMN in the yeast powder by at least 1.1 times. In some embodiments, the yeast powder prepared by the fermentation medium which further contains the *Aronia melanocarpa* extract can increase the content of NMN in the yeast powder by at least 1.47 times. In some embodiments, the yeast powder prepared by the fermentation medium which further contains the *Solanum lycopersicum* 'Kumato' extract can increase the content of NMN in the yeast powder by at least 1.19 times.

Therefore, the yeast powder rich in NMN, prepared by the preparation method of any embodiment, can increase the content of NMN, and in addition, the following multiple embodiments show that the yeast powder rich in NMN can be used for improvement of skin conditions, hair care, anti-inflammation, cardiovascular health care, antioxidation, antiaging, and/or improvement of body fatigue. Moreover, the yeast powder can be used for preparing a composition for improvement of skin conditions, hair care, anti-inflammation, cardiovascular health care, antioxidation, antiaging, and/or improvement of body fatigue.

In some embodiments, the yeast powder can enhance the skin tightness of a subject, reduce wrinkles, reduce the skin roughness of the subject or achieve combinations thereof, so as to improve the skin conditions of the subject. For example, after the subject takes the yeast powder, the skin wrinkles and skin textures of the subject can be reduced, and the skin tightness of the subject can be enhanced to make the skin of the subject smooth and elastic, thereby improving the overall skin condition. Therefore, the yeast powder has the function of improving skin conditions.

In some embodiments, the yeast powder can reduce the hair loss of the subject or/and reduce the degree of hair scantiness of the subject so as to achieve the effect of nursing hair. For example, after the subject takes the yeast powder, the hair loss condition of the subject can be reduced, and the degree of hair scantiness of the subject can be changed. Therefore, the yeast powder has the function of hair care.

In some embodiments, the yeast powder can reduce the content of reactive oxygen species (hereinafter referred to as ROS) in cells. For example, after the subject takes the yeast powder, the cells of the subject can resist the cell oxidation pressure, thereby achieving the effect of antioxidation.

In some embodiments, the yeast powder can activate the activity of mitochondria. For example, after the subject takes the yeast powder, the activity of mitochondria of cells of the subject can be activated, thereby achieving the effect of antioxidation and/or antiaging.

In some embodiments, the yeast powder can achieve the antiaging function by regulating the expression of anti-aging related genes of the subject, activating mitochondria or reducing brain age. For example, the anti-aging genes include *CCT* genes, *PARP2* gene (Gene ID: 10038), *Parkin* gene (Gene ID: 5071), *Atg* genes, *FOXO* gene (Gene ID: 2308), *SIRT1* gene (Gene ID: 23411), *NADSYN* gene (Gene ID: 55191), *MRPS5* gene (Gene ID: 64969), *SOD3* gene (Gene ID: 6649), or combinations thereof. Herein, the *CCT* genes include *CCT5* gene (Gene ID: 22948), *CCT6A* gene (Gene ID: 908), *CCT7* gene (Gene ID: 10574) and *CCT8* gene (Gene ID: 10694). The Atg genes include *Atg1* gene (Gene ID: 8408) and *Atg8* gene (Gene ID: 11345).

In some embodiments, after the subject takes the yeast powder, the expression of the *CCT* gene, the *Parkin* gene, the *Atg* gene, the *FOXO* gene, the *SIRT1* gene, the *NADSYN* gene, the *MRPS5* gene, the *SOD3* gene or the combination thereof can be increased, and the expression of the *PARP2* gene can be inhibited. For example, by increasing the expression of the *CCT* gene which is chaperonin, the correction of the misfolded protein is facilitated, and the protein which cannot be successfully repaired is delivered into proteasome for hydrolysis, thereby avoiding cell function decline and avoiding acceleration of cell aging and death so as to achieve the cell regulating effect of resisting aging. By increasing the expression of the *Parkin* gene, mature cells can be facilitated to return to young cells. By inhibiting the expression of the *PARP2* gene, telomerase activity and cell rejuvenation can be facilitated, and it is ensured that telomerase is not lost at the end of DNA during replication, thereby reducing the probability of cell aging. By increasing the expression of the *Atg* gene and the *MRPS5* gene, the anti-aging ability of cells can be improved, and the service life of cells can be prolonged. By increasing the expression of the *FOXO* gene, aging cells can be removed to protect young cells. By increasing the expression of the *SIRT1* gene, important metabolic functions of the body can be regulated. By increasing the expression of the *NADSYN* gene, the effect of antioxidation is achieved.

In some embodiments, after the subject supplements NMN by taking the yeast powder, the expression of downstream regulatory genes (such as the *SIRT1* gene and the *SOD3* gene) of nicotinamide adenine dinucleotide (NAD⁺) in the blood of the subject is increased, which indicates that the yeast powder can activate mitochondria of subject cells and has an anti-aging ability.

In some embodiments, the yeast powder is used for reducing the expression level of CRP of the subject. The volume of CRP in blood will increase due to the damage of body tissues, chronic inflammation and other conditions, so CRP can be used as a risk index for inflammatory reactions and cardiovascular diseases. For example, after the subject takes the yeast powder, the content of CRP in the blood of the subject can be reduced, which indicates that the risk of cardiovascular diseases of the subject is reduced. Therefore, the yeast powder has the anti-inflammatation function.

In some embodiments, the yeast powder can be used for regulating blood fat. Herein, the regulating blood fat refers to inhibiting the content of low density lipoprotein cholesterol (LDL-C) and reducing the ratio of LDL-C to high density lipoprotein cholesterol (HDL-C) (T.CHOL/HDL). For example, after the subject supplements NMN by taking the yeast powder, the content of LDL-C in the blood of the subject is reduced, and the ratio of LDL-C to HDL-C is reduced, which indicates that the risk of coronary arteriosclerosis of the subject is reduced. Therefore, the yeast powder is used for regulating blood fat and has the function of cardiovascular health care.

In some embodiments, the yeast powder can reduce the brain age. For example, after the subject takes the yeast powder, the brain detection age can be reduced by at least 10 years, which indicates that when the brain age is detected, the brain of the subject is clearer, and the brain is younger. Therefore, the yeast powder has the antiaging function.

In some embodiments, the yeast powder can be used for improvement of body fatigue of the subject. For example, after the subject takes the yeast powder, the body fatigue is effectively reduced.

In some embodiments, the yeast powder can be redissolved in a lysis buffer to react for 10 min with DNase of which the concentration is 1 µg/mL, so as to decompose DNA of the yeast powder. Then, a high-pressure yeast breaker is used for breaking the yeast three times under the pressure values of 25 Kpsi, 30 Kpsi and 32 Kpsi in sequence, so as to form a yeast extract, where the yeast extract contains at least 5000 ppm of NMN. In some embodiments, the yeast extract further includes Q₁₀ biosynthetic protein (COQ protein) and coenzyme Q₁₀. Moreover, since the raw material of the yeast extract is the yeast powder, the yeast extract also has the functions of the above yeast powder for improvement of skin conditions, hair care, anti-inflammation, cardiovascular health care, antioxidation, antiaging, and/or improvement of body fatigue.

Herein, the "yeast extract" refers to substances contained in the protein of the yeast and the raw material (yeast powder) thereof, and the other substances contained in the raw material (yeast powder) may be the metabolites and media of the yeast.

Therefore, the yeast powder rich in NMN prepared by the preparation method of any embodiment, and/or the yeast extract prepared from the yeast powder can be used for preparing a composition for improvement of skin conditions, hair care, anti-inflammation, cardiovascular health care, antioxidation, antiaging, and/or improvement of body fatigue. Herein, the composition at least contains 5000 ppm of NMN. In some embodiments, the composition further includes Q₁₀ biosynthetic protein (COQ protein).

In some embodiments, the composition may be solid (such as powder, tablets and capsules). In some embodiments, the daily dose of the yeast powder is 100 mg. In other words, the dose of the composition is 100 mg of the yeast powder per day.

In some embodiments, any one of the above compositions may be a pharmaceutical product. In other words, the pharmaceutical product contains an effective content of the yeast powder and/or the yeast extract prepared from the yeast powder.

In some embodiments, the above pharmaceutical products can be prepared into enterally, parenterally, orally or topically administrated dosage forms by using the technology known to those skilled in the art.

In some embodiments, the enterally or orally administrated dosage forms may be, but are not limited to, tablets, troches, lozenges, pills, capsules, dispersible powder or granules, solutions, suspensions, emulsions, syrup, elixirs, slurry, or the like. In some embodiments, the parenterally or topically administrated dosage forms may be, but are not limited to, injections, sterile powder, external preparations, or the like. In some embodiments, the administration method of injections may be subcutaneous injection, intraepidermal injection, intradermal injection or intralesional injection.

In some embodiments, the above pharmaceutical products may contain pharmaceutically acceptable carriers which are widely used in pharmaceutical manufacturing technologies. In some embodiments, the pharmaceutically acceptable carrier may be one or more of the following: solvents, buffers, emulsifiers, suspending agents, decomposers, disintegrating agents, dispersing agents, binding agents, excipients, stabilizing agents, chelating agents, diluents, gelling agents, preservatives, wetting agents, lubricants, absorption delaying agents, liposomes, and the like. The types and quantity of the selected carriers fall within the scope of the professional quality and routine technology of those who are familiar with this technology. In some embodiments, a solvent used as a pharmaceutically acceptable carrier may be water, normal saline, phosphate buffered saline (PBS), or an aqueous solution containing alcohol.

In some embodiments, any one of the above compositions may be an edible product. In other words, the edible product contains a specific content of the yeast powder and/or the yeast extract prepared from the yeast powder. In some embodiments, the edible products may be general foods, health foods or dietary supplements.

In some embodiments, the above edible products can be prepared into orally administrated dosage forms by using the technology known to those skilled in the art. In some embodiments, the above general foods may be the edible products. In some embodiments, the general foods may be, but are not limited to, beverages, fermented foods, bakery products or condiments.

In some embodiments, the obtained yeast powder and/or the yeast extract prepared from the yeast powder can be further used as a food additive to prepare a food composition containing the yeast powder and/or the yeast extract prepared from the yeast powder. Herein, the yeast powder of any embodiment can be added during preparation of raw materials by a conventional method, or the yeast powder of any embodiment can be added during preparation of foods, so as to prepare edible products (food compositions) for people and animals together with any edible material.

### Example 1: Fermentation process-Preparation of yeast powder rich in NMN

Fermentation media were prepared and divided into a first medium, a second medium and a third medium according to a fermentation sequence. Herein, the fermentation medium was composed of a basal medium, NAM, tryptophan and niacin. The components of the fermentation medium include 2.0% of yeast peptone GLS (English trade name: Yeast peptone GLS; manufacturer: STBIO MEDIA, INC..), 2.0% of Angel yeast extract 902 (English trade name: Angel yeast extract 902; manufacturer: De'en Biomedical Co., Ltd.), 0.5% of potassium dihydrogen phosphate (Manufacturer: Sigma-Aldrich), 0.5% of potassium hydrogen phosphate (Manufacturer: Zimi Chemicals Co., Ltd.), 0.05% of magnesium sulfate (Manufacturer: Grand union Industrial Co., Ltd.), 10% of glucose (Manufacturer: Lynnbros Industrial Co., Ltd.), 0.17% of citric acid (Manufacturer: Union Food Co., Ltd.), 0.5% of sodium acetate (Manufacturer: Union Food Co., Ltd.), 0.016% of manganese gluconate (Manufacturer: Maylong Trading Co., Ltd.), 0.1% of cysteine (Manufacturer: Lynnbros Industrial Co., Ltd.), 0.05% of defoamer (Manufacturer: HEALTHCARE PLUS Co., Ltd.), 0.1% of NAM (Manufacturer: Sigma-Aldrich), 0.2% of tryptophan (Manufacturer: Sigma-Aldrich), 0.0369% of niacin (Manufacturer: Sigma-Aldrich), and deionized distilled water supplemented to 100%, where the glucose was sterilized separately from other components. Herein, the volume of the first medium was 3 L, the volume of the second medium was 50 L, and the volume of the third medium was 500 L.

3 L of the sterilized first medium was placed in a 5 L fermentation tank, where subsequent strain inoculation can be performed when the DO of the first medium was greater than 40%. Moreover, the Saccharomyces cerevisiae TCI907 was activated first to enable the OD₆₀₀ of the Saccharomyces cerevisiae TCI907 to be 6-8, then, the Saccharomyces cerevisiae TCI907 was inoculated into the first medium with an oxygen content greater than 40% at an inoculum amount of 5 vol% (150 mL) for the first fermentation to form a first fermentation broth, and the fermentation time was 4-5 h. During the fermentation of the first fermentation broth, the fermentation temperature was 30±1°C, the pH value was 6.5±0.1, and the DO value was maintained at 40-50 mg/L.

After the fermentation of the first fermentation broth, the OD₆₀₀ was 4.0-5.0. Then, the first fermentation broth was inoculated into a 75 L fermentation tank containing 50 L of the second medium at an inoculum amount of 6 vol% (3 L) for the second fermentation to form a second fermentation broth, and the fermentation time was 4-5 h, where the DO of the second medium needs to be greater than 40%. Moreover, during the fermentation of the second fermentation broth, the fermentation temperature was 30±1°C, the pH value was 6.5±0.1, and the DO value was maintained at 40-50 mg/L.

After the fermentation of the second fermentation broth, the OD₆₀₀ was 4.0-5.0. Then, the second fermentation broth was inoculated into a 750 L fermentation tank containing 500 L of the third medium at an inoculum amount of 10 vol% (50 L) for the third fermentation to form a third fermentation broth, and the fermentation time was 12-14 h, where the DO of the third medium needs to be greater than 40%. Moreover, during the fermentation of the third fermentation broth, the fermentation temperature was 30±1°C, the pH value was 6.5±0.1, and the DO value was maintained at 40-50 mg/L.

After the fermentation of the third fermentation broth, the OD₆₀₀ was 48.0-53.0. Then, the third fermentation broth was transferred into a centrifuge to obtain a fermented product of which the solid content was greater than 65% and less than 80% and the OD₆₀₀ was greater than 500. Moreover, 40 g of skim milk powder, 120 g of maltodextrin, 80 g of sucrose and 40 g of glycerol were respectively added to each kilogram of the fermented product as a freeze-drying protective agent, and the yeast powder was obtained by freeze-drying and pulverizing.

### Example 2: Fermentation process-Yeast strain test

Herein, NMN in the yeast powder was used as a judgment index.

An experimental group uses the yeast powder prepared in Example 1, and the yeast strain used was the Saccharomyces cerevisiae TCI907.

The yeast powder in a contrast group was prepared according to the preparation process of Example 1, but the strain used in Example 1 was replaced. In other words, the difference between the preparation process of the contrast group and the preparation process of Example 1 was that the yeast strain used was Cyberlindnera jadinii (purchased from the Bioresource Collection and Research Center of the Food Industry Research and Development Institute in Taiwan, China, Deposit No.: BCRC20325).

1 g of the yeast powder was taken from the experimental group and the contrast group respectively and dissolved in 9 g of water respectively to form yeast solutions, and yeast cells were crushed with a cell crusher (Brand: Sunway Scientific Co., Ltd.) on ice. Then, the yeast solutions after cell crushing in the experimental group and the contrast group and a reaction solution were respectively added to a 96-well dish, reaction was performed for 2 min on the ice, then 88% of formic acid (commercially available product) was added, and reaction was performed again at 37°C for 10 min to obtain solutions to be tested. Herein, the reaction solution includes 20% of acetophenone (commercially available product) dissolved in DMSO and 2 M of potassium hydroxide (commercially available product).

Commercially available NMN (Manufacturer: Wuxi Cima Science Co., Ltd.) was sequentially diluted respectively into a plurality of NMN samples with different concentrations such as 0.04 mm to 0.000625 mm, and 250 µL of the NMN samples were respectively taken and added to the 96-well dish, so as to facilitate the subsequent manufacturing of an NMN concentration standard line.

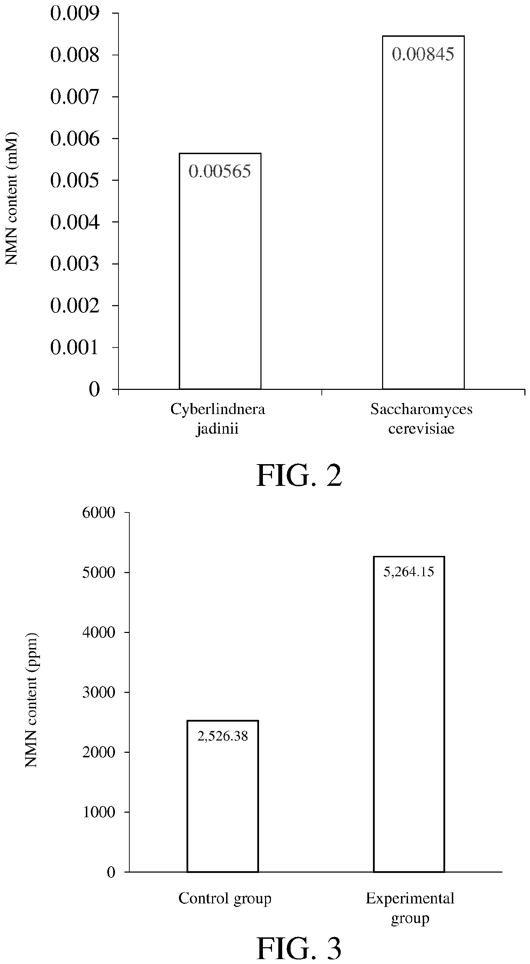

Then, for the solutions to be tested in the experimental group and the contrast group and the plurality of NMN samples with different concentrations, UV rays were measured at 445 nm with the excitation wavelength of 382 nm by a microplate reader (Manufacturer: Thermo Fisher Scientific), corresponding readings were obtained, and the content of NMN in the experimental group and the contrast group was converted by an interpolation method according to the NMN concentration standard line manufactured by the plurality of NMN samples with different concentrations, as shown in FIG. 2.

Referring to FIG. 2, the content of NMN in the experimental group was 0.00845 mm, and the content of NMN in the contrast group was 0.00565 mm. In other words, the content of NMN in the yeast powder prepared from the Saccharomyces cerevisiae in the experimental group was 1.5 times that of the contrast group.

Therefore, the content of NMN in the yeast powder can be increased by using the Saccharomyces cerevisiae for fermentation.

### Example 3: Fermentation process-Fermentation medium test

Herein, NMN in the yeast powder was used as a judgment index.

An experimental group uses the yeast powder prepared in Example 1, and the components of the fermentation medium used include 99.6631% of basal medium, 0.1% of NAM, 0.2% of tryptophan and 0.0369% of niacin, where the components of the basal medium include 2.0% of yeast peptone GLS, 2.0% of yeast extract 902, 0.5% of potassium dihydrogen phosphate (KH₂PO₄), 0.5% of potassium hydrogen phosphate (KH₂PO₄), 0.05% of MgSO₄·7H₂O, 10% of glucose, 0.17% of citric acid, 0.5% of sodium acetate, 0.016% of manganese gluconate, 0.1% of cysteine, 0.05% of defoamer, and deionized distilled water (supplemented to 99.6631%).

The yeast powder in a contrast group was prepared according to the preparation process of Example 1, but the fermentation media used in Example 1 were replaced. In other words, the difference between the preparation process of the contrast group and the preparation process of Example 1 is that the fermentation medium used is the basal medium, and the components of the basal medium include 2.0% of yeast peptone GLS, 2.0% of yeast extract 902, 0.5% of potassium dihydrogen phosphate (KH₂PO₄), 0.5% of potassium hydrogen phosphate (KH₂PO₄), 0.05% of MgSO₄·7H₂O, 10% of glucose, 0.17% of citric acid, 0.5% of sodium acetate, 0.016% of manganese gluconate, 0.1% of cysteine, 0.05% of defoamer, and deionized distilled water (supplemented to 100%).

1 g of the yeast powder was taken from the experimental group and the contrast group respectively and dissolved in 9 g of water respectively to form yeast solutions, and yeast cells were crushed with a cell crusher (Brand: Sunway Scientific Co., Ltd.) on ice. Then, the yeast solutions after cell crushing in the experimental group and the contrast group and a reaction solution were respectively added to a 96-well dish, reaction was performed for 2 min on the ice, then 88% of formic acid (commercially available product) was added, and reaction was performed again at 37°C for 10 min to obtain solutions to be tested. Herein, the reaction solution included 20% of acetophenone (commercially available product) dissolved in DMSO and 2 M of potassium hydroxide (commercially available product).

Commercially available NMN (Manufacturer: Wuxi Cima Science Co., Ltd.) was sequentially diluted respectively into a plurality of NMN samples with different concentrations such as 0.04 mm to 0.000625 mm, and 250 µL of the NMN samples were respectively taken and added to the 96-well dish, so as to facilitate the subsequent manufacturing of an NMN concentration standard line. Moreover, the concentration was converted to ppm.

Then, for the solutions to be tested in the experimental group and the contrast group and the plurality of NMN samples with different concentrations, UV rays were measured at 445 nm with the excitation wavelength of 382 nm by a microplate reader (Manufacturer: Thermo Fisher Scientific), corresponding readings were obtained, and the content of NMN in the experimental group and the contrast group was converted by an interpolation method according to the NMN concentration standard line manufactured by the plurality of NMN samples with different concentrations, as shown in FIG. 3.

Referring to FIG. 3, the content of NMN in the experimental group was 5264.15 ppm, and the content of NMN in the contrast group was 2526.38 ppm. In other words, the content of NMN in the yeast powder prepared from the Saccharomyces cerevisiae in the experimental group was 2.1 times that of the contrast group.

Therefore, the content of NMN in the yeast powder can be increased by using the fermentation medium containing NAM, tryptophan and niacin for fermentation.

### Example 4: Fermentation process-Fermentation medium test (plant extracts and compounds)

Herein, NMN in the yeast powder was used as a judgment index, and the content of NMN in the yeast of Example 1 was taken as an index basis, so as to judge the relative content ratio of NMN in each group (hereinafter referred to as the relative content ratio of NMN).

A control group uses the yeast powder prepared in Example 1, and the components of the fermentation medium used include 99.6631% of basal medium, 0.1% of NAM, 0.2% of tryptophan and 0.0369% of niacin. The components of the basal medium were the same as those recorded in Example 1 and Example 2, so the components of the basal medium were not repeated.

Experimental groups include a *Musa spp.* peel extract group, an *Aronia melanocarpa* extract group and a *Solanum lycopersicum* 'Kumato' extract group, where the yeast powder in the experimental groups was prepared according to the preparation process of Example 1, but the fermentation media used in Example 1 were replaced. In other words, the difference between the preparation process of the experimental groups and the preparation process of Example 1 is that the fermentation medium used contains 0.1% of NAM, 0.2% of tryptophan, 0.0369% of niacin and corresponding percentages of plant extracts. Moreover, the plant extract used in the *Musa spp.* peel extract group was 0.05% of *Musa spp.* peel extract, the plant extract used in the Aronia melanocarpa extract group was 0.085% of *Aronia melanocarpa* extract, the plant extract used in the *Solanum lycopersicum* 'Kumato' extract group was 0.05% of *Solanum lycopersicum* 'Kumato' extract, and the basal medium was supplemented to 100%.

Herein, the *Musa spp.* peel extract used in the *Musa spp.* peel extract group can be provided by separating peel from pulp of *Musa spp.* and performing the following operations on *Musa spp.* peel: 1. the *Musa spp.* peel was mixed with an extraction solvent (the volume ratio of the *Musa spp.* peel to the extraction solvent was 1:6, where the extraction solvent was provided in the volume ratio of citric acid:water=1:100), and extraction was performed at 85°C for 0.5 h to provide a crude extract; 2. the crude extract in step 1 was taken and centrifuged at the rotating speed of 5000 rpm for 10 min, and a supernatant was taken and then filtered with a 400-mesh filter screen to provide a filtrate; 3. at 55±5°C, the filtrate in step 2 was concentrated under reduced pressure to provide a concentrated extract; and 4. the concentrated extract in step 3 was freeze-dried to provide a dried product (that is, the *Musa spp.* peel extract). The *Aronia melanocarpa* extract used in the *Aronia melanocarpa* extract group was obtained by cutting fruits of *Aronia melanocarpa* into pieces and homogenizing and grinding the pieces to form an *Aronia melanocarpa* homogenate, and then extracting the *Aronia melanocarpa* homogenate with water at 40-60°C for 0.5-2 h, where the liquid-solid ratio of the water to the *Aronia melanocarpa* homogenate was (5-20):(1-5). The *Solanum lycopersicum* 'Kumato' extract used in the Solanum lycopersicum 'Kumato' extract group was obtained by cutting *Solanum lycopersicum* 'Kumato' into pieces and homogenizing and grinding the pieces to form a *Solanum lycopersicum* 'Kumato' homogenate, and then extracting the *Solanum lycopersicum* 'Kumato' homogenate with water at 40-60°C for 0.5-2 h, where the liquid-solid ratio of the water to the *Solanum lycopersicum* 'Kumato' homogenate was (5-20):(1-5).

Contrast groups include a tannic acid group, a licorice extract group and a passionflower extract group, where the yeast powder in the contrast groups was prepared according to the preparation process of Example 1, but the fermentation media used in Example 1 were replaced. In other words, the difference between the preparation process of the contrast groups and the preparation process of Example 1 is that the fermentation medium used contains 0.1% of NAM, 0.2% of tryptophan, 0.0369% of niacin and corresponding percentages of plant extracts or compounds. Moreover, the compound used in the tannic acid group was 5 µm of tannic acid (Manufacturer: Sigma-Aldrich), the plant extract used in the licorice extract group was 0.5% of licorice extract, the plant extract used in the passionflower extract group was 4% of passionflower extract, and the basal medium was supplemented to 100%.

Herein, the licorice extract used in the licorice extract group was obtained by chopping the licorice root, uniformly mixing the chopped licorice root with aqueous solutions containing 0.5-2% of citric acid respectively in a solid-liquid ratio of (1:2)-(1:10), extracting the mixed solution at 50-100°C for 30-120 min, then cooling to room temperature, and filtering with a 200 µm filter screen. The passionflower extract used in the passionflower extract group was prepared by cleaning and drying the seeds of *Passiflora edulis* (*Passiflora spp.* plant), then coarsely crushing the seeds by a homogenizer to form a passionflower seed homogenate, mixing the passionflower seed homogenate with water in a material-water ratio (weight) of 1:5, extracting at 55°C for 1 h, and then extracting at 85°C for 1 h.

1 g of the yeast powder was taken from the control group, the experimental groups and the contrast groups respectively and dissolved in 9 g of water respectively to form yeast solutions, and yeast cells were crushed with a cell crusher (Brand: Sunway Scientific Co., Ltd.) on ice. Then, the yeast solutions after cell crushing in the control group, the experimental groups and the contrast groups and a reaction solution were respectively added to a 96-well dish, reaction was performed for 2 min on the ice, then 88% of formic acid (commercially available product) was added, and reaction was performed again at 37°C for 10 min to obtain solutions to be tested. Herein, the reaction solution includes 20% of acetophenone (commercially available product) dissolved in DMSO and 2 M of potassium hydroxide (commercially available product).

Commercially available NMN (Manufacturer: Wuxi Cima Science Co., Ltd.) was sequentially diluted respectively into a plurality of NMN samples with different concentrations such as 0.04 mm to 0.000625 mm, and 250 µL of the NMN samples were respectively taken and added to the 96-well dish, so as to facilitate the subsequent manufacturing of an NMN concentration standard line.

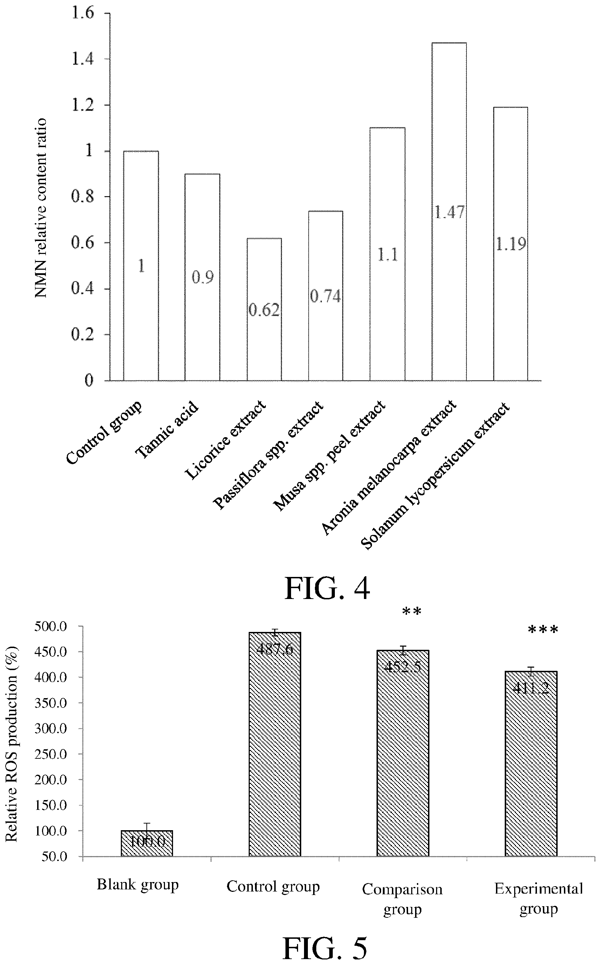

Then, for the solutions to be tested in the control group, the experimental groups and the contrast groups and the plurality of NMN samples with different concentrations, UV rays were measured at 445 nm with the excitation wavelength of 382 nm by a microplate reader (Manufacturer: Thermo Fisher Scientific), corresponding readings were obtained, and the content of NMN in the control group, the experimental groups and the contrast groups was converted by an interpolation method according to the NMN concentration standard line manufactured by the plurality of NMN samples with different concentrations. Herein, the reading obtained by the control group was regarded as 1 to convert the relative content ratio of NMN in the experimental groups and the contrast groups, as shown in FIG. 4.

Referring to FIG. 4, the relative content ratio of NMN in the control group was regarded as 1.0. Compared with the control group, the relative content ratio of NMN in the tannic acid group in the contrast groups was 0.9, the relative content ratio of NMN in the licorice extract group was 0.62, and the relative content ratio of NMN in the passionflower extract group was 0.74. Compared with the control group, the relative content ratio of NMN in the *Musa spp.* peel extract group in the experimental groups was 1.1, the relative content ratio of NMN in the *Aronia melanocarpa* extract group was 1.47, and the relative content ratio of NMN in the *Solanum lycopersicum* 'Kumato' extract group was 1.19. In other words, compared with the control group and the contrast groups, the yeast powder in each group, prepared by the fermentation medium added with 0.05% of the *Musa spp.* peel extract, 0.085% of the *Aronia melanocarpa* extract and 0.05% of the *Solanum lycopersicum* 'Kumato' extract respectively, has higher content of NMN. Moreover, when the fermentation medium was added with 0.085% of the *Aronia melanocarpa* extract, the content of NMN in the prepared yeast powder was close to 1.5 times. In addition, it can be seen from the experimental results of the contrast groups that not all plant extracts can increase the content of NMN in the yeast powder.

It can be seen that when the fermentation medium was added with the *Musa spp.* peel extract, the *Aronia melanocarpa* extract and the *Solanum lycopersicum* 'Kumato' extract, the content of NMN in the yeast powder prepared by the fermentation medium can be increased.

### Example 5: Cell experiment-Inhibition of ROS production (hydrogen peroxide treatment)

Herein, the change of the content of ROS in mouse brain neuroblastoma cells (Neuro2a) after being treated with the yeast powder containing NMN was measured by a fluorescent probe DCFH-DA combined with flow cytometry.

The medium used was DMEM (Dulbecco's Modified Eagle Medium, Brand: Gibco) added with 10 vol% of fetal bovine serum (FBS, Brand: Gibco) and 1 vol% of penicillin/streptomycin (Brand: Gibco), hereinafter referred to as cell medium. The DCFH-DA solution used was prepared by dissolving 2,7-dichloro-dihydro-fluorescein diacetate (DCFH-DA, Product No.: SI-D6883, purchased from Sigma) in dimethyl sulfide (DMSO, purchased from Sigma, Product No.: SI-D6883-50MG), and the reaction was easy.

First, 2×10⁵ mouse neuroblastoma cells (ATCC CCL-131, hereinafter referred to as Neuro2a cells) were taken and cultured at 37°C for 24 h in a six-well cell culture dish containing 2 mL of the cell medium per well.

After the Neuro2a cells from each well were respectively attached to the bottom of the six-well cell culture dish, the Neuro2a cells were divided into a blank group, a control group, a contrast group and an experimental group. Subsequently, the cell medium in each group was replaced with an experimental medium, and then, the experimental medium was continuously cultured at 37°C for 24 h. The experimental media in the blank group and the control group were pure cell media. The experimental medium in the contrast group was a cell medium added with 10 µL of chemically synthesized NMN (Manufacturer: Sigma-Aldrich) with a concentration of 200 mm. The experimental medium in the experimental group was a cell medium containing 0.25 vol% of the yeast powder rich in NMN prepared in Example 1.

Then, 2 µL of 5 µg/mL DCFH-DA solution was added to each group, and the Neuro2a cells were treated for 15 min in the experimental medium of each well. After the DCFH-DA solution reacts, 10 µL of 200 mm hydrogen peroxide (Sigma-Aldrich) was added to the experimental medium in each group, and reacts at 37°C for 1 h.

Subsequently, after the experimental medium in each group was removed, the Neuro2a cells in each group were rinsed twice with 1 mL of 1X PBS solution. Then, 200 µL of trypsin was added to each well to react for 5 min. After the reaction, 1 mL of the cell medium was added to each well to terminate the reaction. The Neuro2a cells and cell medium in each well were collected into individual corresponding 1.5 mL microcentrifuge tubes, and the microcentrifuge tubes containing the Neuro2a cells and cell medium were centrifuged at 400 xg for 5 min. The supernatant in the microcentrifuge tube in each group was removed after centrifugation, and the precipitates of the Neuro2a cells were redissolved with 1X PBS solution and centrifuged at 400 xg for 5 min. The supernatant in the microcentrifuge tube in each group was removed again after centrifugation, and the Neuro2a cells were suspended again in the dark with 200 µL of 1X PBS solution per centrifuge tube, so as to obtain the cell sap to be detected in each group.

The fluorescence signal of DCFH-DA in the cell sap to be detected in each group was detected by a flow cytometry (Manufacturer: Beckman, Catalog No.660519). The excitation wavelength of the fluorescence detection used was 450-490 nm, and the emission wavelength was 510-550 nm. Since after entering the Neuro2a cells, DCFH-DA was first hydrolyzed into DCFH (dichlorodihydrofluorescein) and then oxidized by ROS to DCF (dichlorofluorescein) which can emit green fluorescence, the fluorescence intensity of the Neuro2a cells treated with DCFH-DA can reflect the content of ROS in the Neuro2a cells, so as to know the proportion of the number of cells highly expressing ROS in the Neuro2a cells to the number of protocells. Since the experiment was repeated twice, the measurement results of the two repeated experiments in each group were averaged to obtain an average value, and then, the average values of the control group, the contrast group and the experimental group were converted into the output relative to ROS by taking the average value of the blank group as 100% output relative to ROS, as shown in FIG. 5. Moreover, the expression of the Neuro2a cells in each group was observed by a fluorescence microscope (Beckman).

Referring to FIG. 5, the cells in the blank group were not treated with hydrogen peroxide, the output relative to ROS in the blank group was used as 100% reference, and under the fluorescence microscope, the Neuro2a cells in the blank group show blue, indicating that there was no induced cell oxidation pressure. After the control group was treated with hydrogen peroxide, the output relative to ROS (high fluorescence expression) in the control group was greatly increased by 487.6% compared with the blank group, and under the fluorescence microscope, the Neuro2a cells in the control group show fluorescent green, indicating that hydrogen peroxide treatment can indeed lead to production of ROS in cells, thus causing subsequent damage to the Neuro2a cells. After the contrast group was treated with hydrogen peroxide, the output relative to ROS in the contrast group was increased by 452.5% compared with the blank group but reduced by 35.1% compared with the control group. Moreover, under the fluorescence microscope, the Neuro2a cells in the contrast group show bluegreen interlaced color but green was relatively obvious, indicating that the chemically synthesized NMN in the cell medium can slightly help the Neuro2a cells resist the cell oxidation pressure caused by hydrogen peroxide. After the experimental group was treated with hydrogen peroxide, the output relative to ROS in the experimental group was increased by 411.2% compared with the blank group but reduced by 76.4% compared with the control group. Moreover, under the fluorescence microscope, the Neuro2a cells in the experimental group mainly show blue with slight green, indicating that the natural NMN in the cell medium can effectively reduce the production or accumulation of ROS in cells, thereby better helping the Neuro2a cells resist the cell oxidation pressure caused by hydrogen peroxide. Moreover, compared with the contrast group, the output relative to ROS of the Neuro2a cells in the experimental group was reduced significantly (about 2.17 times), indicating that compared with the chemically synthesized NMN, the natural NMN in the yeast powder can further effectively reduce the production or accumulation of ROS in cells to help the cells resist the damage of ROS, and has the functions of antioxidation and antiaging.

In other words, the yeast powder rich in NMN can be used as a scavenging agent for ROS. That is, the yeast powder rich in NMN reduce the oxidative damage of cells caused by ROS, etc. by reducing the content of ROS in cells.

Therefore, when the subject takes the yeast powder rich in NMN, the damage of ROS can be further effectively resisted, so as to achieve the effects of antioxidation and antiaging.

### Example 6: Cell experiment-Activity of mitochondria

Herein, the change of the activity of mitochondria in mouse brain neuroblastoma cells (Neuro2a) after being treated with the yeast powder containing NMN was evaluated by a flow cytometry.

The medium used was DMEM (Dulbecco's Modified Eagle Medium, Brand: Gibco) added with 10 vol% of fetal bovine serum (FBS, Brand: Gibco) and 1 vol% of penicillin/streptomycin (Brand: Gibco), hereinafter referred to as cell medium. A method used for testing the activity of mitochondria uses a mitochondrial membrane potential detection kit (BDTM MitoScreen (JC-1) kit, Model: 551302) for measuring the membrane potential of mitochondria and performing activity analysis of mitochondria, where the mitochondrial membrane potential detection kit contains a JC-1 dye (freeze-dried) and a 10X analytical buffer. Before use, the 10X analytical buffer was diluted 10 times with 1X PBS to form a 1X analytical buffer. 130 µL of DMSO was added to the JC-1 dye (freeze-dried) to form a JC-1 stock solution. Then, the JC-1 stock solution was diluted with the 1X analytical buffer to form a JC-1 working solution (that is, JC-1 mitochondrial specific dye). The dilution ratio of the JC-1 stock solution to the 1X analytical buffer was 1:100.

First, 1×10⁵ mouse neuroblastoma cells (ATCC CCL-131, hereinafter referred to as Neuro2a cells) were taken and cultured at 37°C for 24 h in a six-well cell culture dish containing 2 mL of the cell medium per well.

After the Neuro2a cells from each well were respectively attached to the bottom of the six-well cell culture dish, the Neuro2a cells were divided into an experimental group, a contrast group and a control group. Subsequently, the cell medium in each group was replaced with an experimental medium, and then, the experimental medium was continuously cultured at 37°C for 24 h. The experimental medium in the experimental group was a cell medium containing 0.25 vol% of the yeast powder prepared in Example 1. The experimental medium in the contrast group was a cell medium added with 0.25 vol% of chemically synthesized NMN (Manufacturer: Sigma-Aldrich). The experimental medium in the control group was a pure cell medium.

Subsequently, after the experimental medium in each group was removed, the Neuro2a cells in each group were rinsed twice with 1 mL of 1X DPBS solution. Then, 200 µL of trypsin was added to each well to react for 5 min. After the reaction, 1 mL of the cell medium was added to each well to terminate the reaction. The Neuro2a cells and cell medium in each well were collected into individual corresponding 1.5 mL microcentrifuge tubes, and the microcentrifuge tubes containing the Neuro2a cells and cell medium were centrifuged at 400 xg for 5 min. The supernatant in the microcentrifuge tube in each group was removed after centrifugation, and the precipitates of the Neuro2a cells were redissolved with 1X DPBS solution and centrifuged at 400 xg for 5 min. The supernatant in the microcentrifuge tube in each group was removed again after centrifugation, 100 µL of the JC-1 working solution was added to each microcentrifuge tube to enable the precipitates of the Neuro2a cells to be uniformly mixed with the JC-1 working solution, and then, the mixture stands for 15 min in a dark place. After standing for 15 min, the microcentrifuge tube containing the Neuro2a cells and JC-1 working solution was centrifuged at 400 xg for 5 min. Then, the supernatant in the microcentrifuge tube in each group was removed, the 1X analytical buffer was added to redissolve the precipitates of the Neuro2a cells, centrifugation was performed at 400 xg for 5 min, and the process was repeated twice. The supernatant in the microcentrifuge tube in each group was removed, and the precipitates of the Neuro2a cells were suspended again with the 1X DPBS solution containing 2 vol% of FBS, so as to obtain the cell sap to be detected.

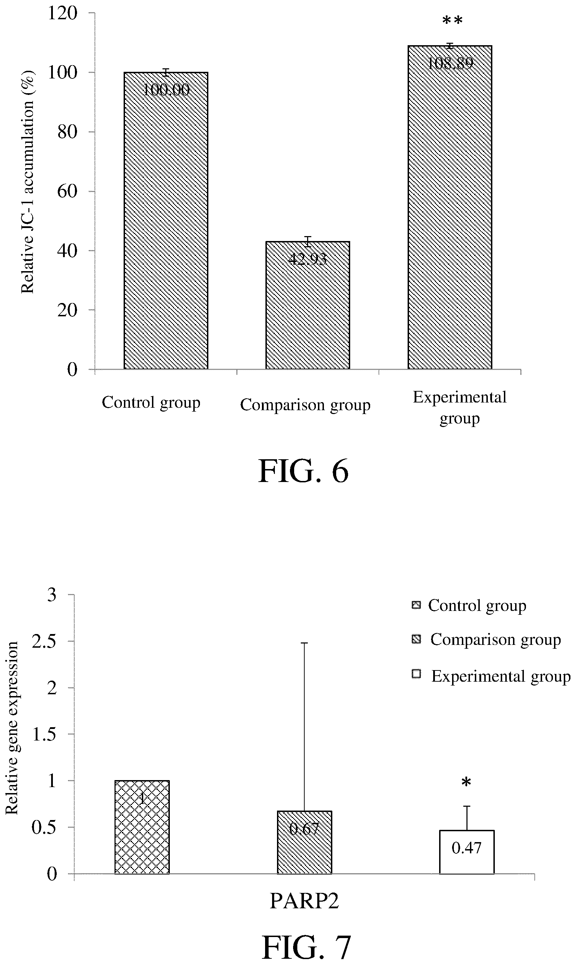

A flow cytometry (Manufacturer: Beckman, Catalog No.660519) was used for measuring the membrane potential of cell mitochondria in the cell sap to be detected in each group, so as to perform activity analysis of mitochondria, where the wavelength of the excitation light, set by the flow cytometry, was 488 nm, and the wavelength of the scattering light was 527 nm and 590 nm. Since the experiment was repeated three times, the results of the three repeated experiments in each group were averaged to obtain an average value, and then, the average relative JC-1 accumulation in the experimental group and the contrast group was converted by taking the average value of the control group as 100% relative JC-1 accumulation, as shown in FIG. 6. In FIG. 6, "**" represents that the p value was less than 0.01 compared with the control group. Moreover, a fluorescence microscope (Beckman) was used for observing the expression of mitochondria of the Neuro2a cells in each group, where green fluorescence represents that cell mitochondria were not activated, and red fluorescence represents that cell mitochondria were activated.

Referring to FIG. 6, the relative JC-1 accumulation of the control group was 100%. Moreover, under the fluorescence microscope, the mitochondria in the Neuro2a cells in the control group show red-green interlaced fluorescence and green fluorescence predominates, indicating that the mitochondria in the Neuro2a cells in the control group were partially activated but mostly were not activated. The relative JC-1 accumulation of the contrast group was 42.93%, and under the fluorescence microscope, the mitochondria in the Neuro2a cells in the contrast group show green fluorescence mainly, indicating that most of the mitochondria in the Neuro2a cells in the contrast group were not activated. The relative JC-1 accumulation of the experimental group was 108.89%, and under the fluorescence microscope, the mitochondria in the Neuro2a cells in the experimental group show red fluorescence mainly, indicating that most of the mitochondria in the Neuro2a cells in the contrast group were activated. In other words, compared with the contrast group, the activity of the mitochondria in the Neuro2a cells in the experimental group was increased significantly (about 1.09 times). Therefore, the yeast powder rich in NMN increase the activity of mitochondria in nerve cells, so as to achieve the effect of increasing the activity of nerve cells. In addition, it can be seen from FIG. 6 that the chemically synthesized NMN cannot activate the activity of mitochondria in nerve cells.

Therefore, when the subject takes the yeast powder rich in NMN, the activity of mitochondria in nerve cells can be further effectively increased, so as to achieve the effects of increasing the activity of nerve cells and resisting aging.

### Example 7: Cell experiment-Anti-aging related genes

Herein, the detected anti-aging related genes were anti-aging genes, including: a CCT5 gene (Gene ID: 22948), a CCT6A gene (Gene ID: 908), a CCT7 gene (Gene ID: 10574), a CCT8 gene (Gene ID: 10694), a PARP2 gene (Gene ID: 10038), a Parkin gene (Gene ID: 5071), an Atg1 gene (Gene ID: 8408), an Atg8 gene (Gene ID: 11345), an FOXO gene (Gene ID: 2308), an SIRT1 gene (Gene ID: 23411), an NADSYN gene (Gene ID: 55191), and an MRPS5 gene (Gene ID: 64969).

The medium used was an X-VIVOTM 15 Serum-free Hematopoietic Cell Medium (Manufacturer: Lonza, Article No.: 04-418Q) added with 10 vol% of fetal bovine serum (FBS, Brand: Gibco) and 1 vol% of penicillin/streptomycin (Brand: Gibco), hereinafter referred to as a cell medium.

First, 1×10⁶ peripheral blood mononuclear cells (PBMC, isolated from the human body, purchased from ATCC, Model: PCS-200-010^{™}, hereinafter referred to as PBMC cells) were taken and cultured at 37°C for 24 h in a six-well cell culture dish containing 2 mL of the cell medium per well.

The PBMC cells were divided into an experimental group, a contrast group and a control group. The cell medium in each group was removed and replaced with 2 mL of the experimental medium for each well, and then, the experimental medium was continuously cultured at 37°C for 24 h respectively. The experimental medium in the experimental group was a cell medium containing 0.25 vol% of the yeast powder prepared in Example 1. The experimental medium in the contrast group was a cell medium added with 0.25 vol% of chemically synthesized NMN (Manufacturer: Sigma-Aldrich). The experimental medium in the control group was a pure cell medium.

PBMC cells in each group were collected, and RNA in each group was extracted with an RNA extraction reagent set (purchased from Geneaid, Taiwan, China, Lot No. FC24015-G). Then, 1000 ng of RNA was taken from each group as a template, and SuperScript^{®} III reverse transcriptase (purchased from Invitrogene, USA, No. 18080-051) was used for reversely transcribing RNA into corresponding cDNA. Then, an ABI StepOnePlusTM Real-Time PCR system (Thermo Fisher Scientific, USA), KAPA SYBR FAST (purchased from Sigma, USA, No. 38220000000) and primers (SEQ ID NO: 1 to SEQ ID NO: 24) in Table 1 were used for performing quantitative real-time reverse transcription polymerase chain reaction on cDNA of each group, so as to observe the expression of anti-aging related genes in the PBMC cells. An instrument for the quantitative real-time reverse transcription polymerase chain reaction was set to react at 95°C for 20s, then react at 95°C for 3s, react at 60°C for 30s and repeat 40 cycles, and a 2-ΔCt method was used for gene quantification, as shown in FIG. 7 to FIG. 10. Therefore, by performing the quantitative real-time reverse transcription polymerase chain reaction through cDNA, the mRNA expression of genes can be quantified indirectly, thereby deducing the expression of protein encoded by genes.

It should be noted that the gene expression in FIG. 7 to FIG. 10 was shown in relative expression ratio, where the STDEV formula of Excel software was used for computing the standard deviation, and the statistically significant differences between groups were statistically analyzed by the student's t-test. In FIG. 7 to FIG. 10, "*" represents that the p value was less than 0.05 compared with the control group, "**" represents that the p value was less than 0.01 compared with the control group, and "***" represents that the p value was less than 0.001 compared with the control group.

**Table 1**

| Target gene | Primer name | Sequence number | Sequence | Primer length |
|---|---|---|---|---|
| PARP2 | PARP2-F | SEQ ID NO: 1 | AGCAAGATGAATCTGTGAAGGC | 22 |
| | PARP2-R | SEQ ID NO:2 | CACTGAAGTTCCTCTGGGCA | 20 |
| CCT5 | CCT5-F | SEQ ID NO:3 | ATAAATGTGAGGCTGAATC | 19 |
| | CCT5-R | SEQ ID NO:4 | ACTTGTCACTTGTGGCAC | 18 |
| CCT6A | CCT6A-F | SEQ ID NO: 5 | TGTGTATCTTAATCCAGACTC | 21 |
| | CCT6A-R | SEQ ID NO:6 | CGTTTCACCTAAGAGTTGTC | 20 |
| CCT7 | CCT7-F | SEQ ID NO:7 | GTGGCATGGACAAGCTTATTGTAG | 24 |
| | CCT7-R | SEQ ID NO: 8 | CAGAATTGTGGCCCCATCA | 19 |
| CCT8 | CCT8-F | SEQ ID NO:9 | ACCCGGAGGTGGAGCAA | 17 |
| | CCT8-R | SEQ ID NO:10 | GGACATGTCTCTCCATATGATGTGA | 25 |
| Parkin | Parkin-F | SEQ ID NO:11 | GCAGAGACCGTGGAGAAAAG | 20 |
| | Parkin-R | SEQ ID NO:12 | CTTTTCTCCACGGTCTCTGC | 20 |
| Atg1 | Atg1-F | SEQ ID NO:13 | CAGGAGGACGAGAACACGGTGTC | 23 |
| | Atg1-R | SEQ ID NO:14 | GGAAGGTTCTTTGGCACCAGCAC | 23 |
| Atg8 | Atg8-F | SEQ ID NO:15 | CCGCAGTAGGTGGCAAAGTA | 20 |
| | Atg8-R | SEQ ID NO:16 | GGAGTCGGAGAGGATTGCTG | 20 |
| FOXO | FOXO-F | SEQ ID NO:17 | CGGACAAACGGCTCACTCT | 19 |
| | FOXO-R | SEQ ID NO:18 | GGACCCGCATGAATCGACTAT | 21 |
| SIRT1 | SIRT1-F | SEQ ID NO:19 | TGCTGGCCTAATAGAGTGGCA | 21 |
| | SIRT1-R | SEQ ID NO:20 | CTCAGCGCCATGGAAAATGT | 20 |
| NADSYN | NADSYN-F | SEQ ID NO:21 | GCAAAATGTGCAGGCTCGAA | 20 |
| | NADSYN-R | SEQ ID NO:22 | GCACTGGAGCAGTCGTACTT | 20 |
| MRPS5 | MRPS5-F | SEQ ID NO:23 | GTCCGGACAGTCCCTCAC | 18 |
| | MRPS5-R | SEQ ID NO:24 | CCCAATAAATGACCTGCCGTC | 21 |

In Table 1, F represents forward primer, and R represents reverse primer.

Referring to FIG. 7, the relative expression ratio of each group of PARP2 genes in the control group was regarded as 1.00 (that is, the expression of each group of genes in the control group was 100%). Compared with the control group, the relative expression ratio of the PARP2 gene in the experimental group was 0.47, and the relative expression ratio of the PARP2 gene in the contrast group was 0.67. Moreover, compared with the contrast group, the expression of the PARP2 gene in the experimental group was reduced significantly, indicating that compared with the chemically synthesized NMN, the yeast powder rich in naturally synthesized NMN further effectively inhibit the expression of the PARP2 gene. In other words, when the subject takes the yeast powder rich in naturally synthesized NMN, the expression of the PARP2 gene is effectively inhibited, thereby facilitating cell rejuvenation and telomerase activity, and achieving the antiaging function.

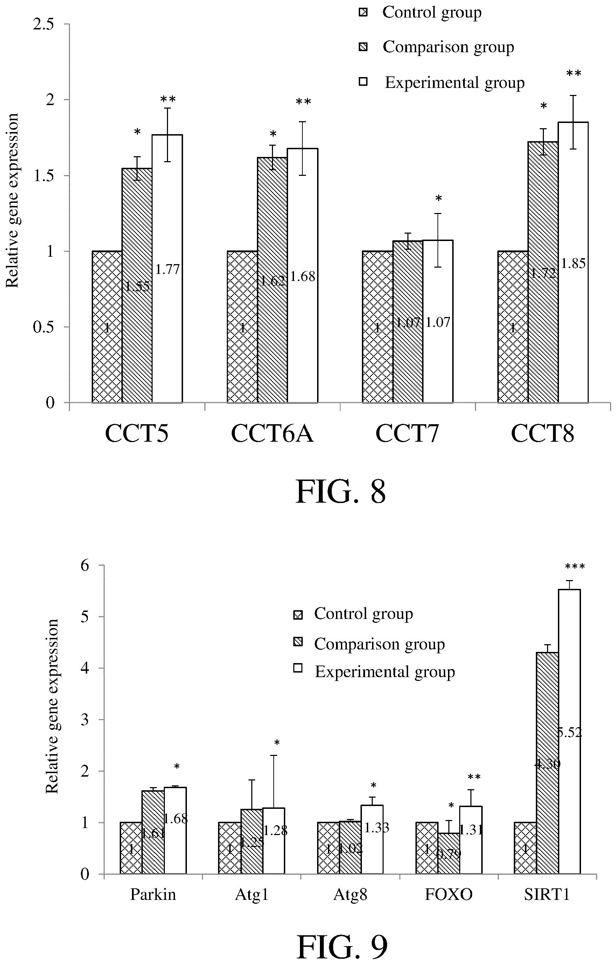

Referring to FIG. 8, the relative expression ratio of each group of CCT genes in the control group was regarded as 1.00 (that is, the expression of each group of CCT genes in the control group was 100%). Compared with the control group, the relative expression ratio of the CCT5 gene in the experimental group was 1.77, the relative expression ratio of the CCT6A gene was 1.68, the relative expression ratio of the CCT7 gene was 1.0736 (1.07 as shown in FIG. 8), and the relative expression ratio of the CCT8 gene was 1.85. The relative expression ratio of the CCT5 gene in the contrast group was 155, the relative expression ratio of the CCT6A gene was 1.62, the relative expression ratio of the CCT7 gene was 1.0672 (1.07 as shown in FIG. 8), and the relative expression ratio of the CCT8 gene was 1.72. It can be seen that the expression of the CCT gene in the experimental group was significantly higher than the expression of the CCT gene in the contrast group, indicating that compared with the chemically synthesized NMN, the yeast powder rich in naturally synthesized NMN further effectively increase the expression of the CCT gene, thereby restoring mature cells to young cells, and achieving the effect of resisting aging. In other words, when the subject takes the yeast powder rich in naturally synthesized NMN, the expression of the CCT gene is effectively increased, thereby facilitating cell rejuvenation, and achieving the antiaging function.

Referring to FIG. 9, the relative expression ratio of each group of anti-aging related genes (herein, referring to *Parkin* gene, *Atg1* gene, *Atg8* gene, *FOXO* gene, *SIRT1* gene) in the control group was regarded as 1.00 (that is, the expression of each group of genes in the control group was 100%). Compared with the control group, the relative expression ratio of the *Parkin* gene in the experimental group was 1.68, the relative expression ratio of the *Atg1* gene was 1.28, the relative expression ratio of the *Atg8* gene was 1.33, the relative expression ratio of the FOXO gene was 1.31, and the relative expression ratio of the SIRT1 gene was 5.52; and the relative expression ratio of the Parkin gene in the contrast group was 1.61, the relative expression ratio of the *Atg1* gene was 1.25, the relative expression ratio of the *Atg8* gene was 1.02, the relative expression ratio of the *FOXO* gene was 0.79, and the relative expression ratio of the *SIRT1* gene was 4.30. Moreover, compared with the contrast group, the expression of the anti-aging related genes in the experimental group was increased significantly, indicating that compared with the chemically synthesized NMN, the yeast powder rich in naturally synthesized NMN further effectively facilitate the expression of the *Parkin* gene, the *Atg1* gene, the *Atg8* gene, the *FOXO* gene and the *SIRT1* gene. In other words, when the subject takes the yeast powder rich in naturally synthesized NMN, the expression of the *Parkin* gene, the *Atg1* gene, the *Atg8* gene, the *FOXO* gene and the *SIRT1* gene can be effectively increased, thereby facilitating cell rejuvenation, and achieving the antiaging function.

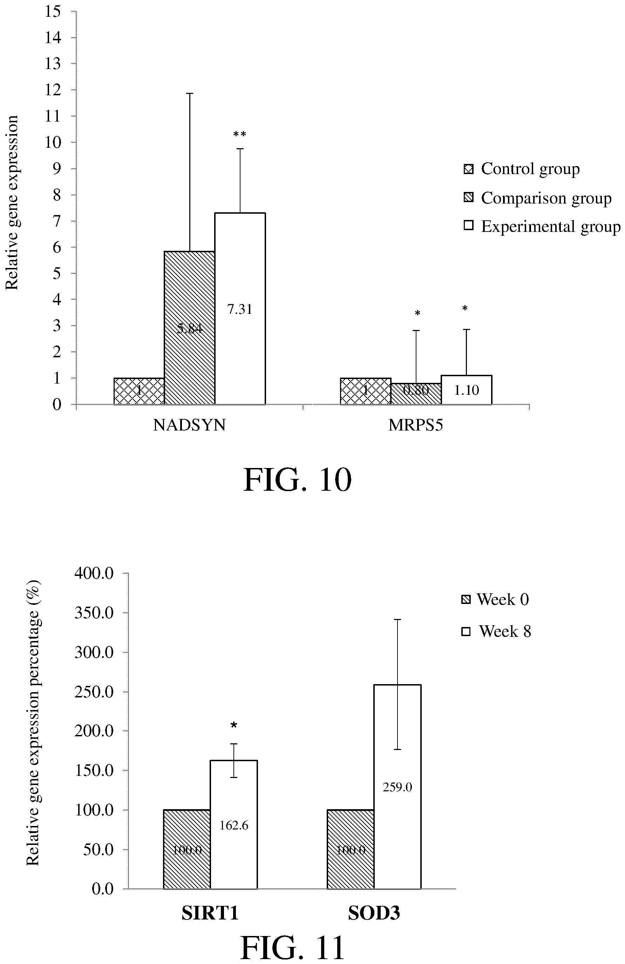

Referring to FIG. 10, the relative expression ratio of each group of NADSYN genes and MRPS5 genes in the control group was regarded as 1.00 (that is, the expression of each group of *NADSYN* genes and *MRPS5* genes in the control group was 100%). Compared with the control group, the relative expression ratio of the *NADSYN* gene in the experimental group was 7.31, and the relative expression ratio of the *MRPS5* gene was 1.10; and the relative expression ratio of the *NADSYN* gene in the contrast group was 5.84, and the relative expression ratio of the *MRPS5* gene was 0.80. It can be seen that the expression of the *NADSYN* gene and the *MRPS5* gene in the experimental group was significantly higher than the expression of the *NADSYN* gene and the *MRPS5* gene in the contrast group, indicating that compared with the chemically synthesized NMN, the yeast powder rich in naturally synthesized NMN further effectively increase the expression of the *NADSYN* gene and the *MRPS5* gene, thereby improving the cell abilities of antiaging and antioxidation. In other words, when the subject takes the yeast powder rich in naturally synthesized NMN, the expression of the *NADSYN* gene and the *MRPS5* gene can be effectively increased, thereby achieving the functions of antioxidation and antiaging, and facilitating cell rejuvenation.

In addition, referring to FIG. 9 and FIG. 10, the expression of the *FOXO* gene and the *MRPS5* gene in the contrast group was lower than that in the control group, indicating that the chemically synthesized NMN inhibit the expression of the *FOXO* gene and the *MRPS5* gene. In other words, not all NMNs facilitate the expression of the anti-aging genes.

### Example 8: Human body test

In order to further determine the influence of the yeast powder rich in NMN on the human body, capsules, each of which contains 100 mg of the yeast powder prepared in Example 1, were provided to 8 subjects, and each subject takes one capsule daily for 8 consecutive weeks, where the eight subjects were healthy men and women aged 50 to 75.

Before taking the capsule containing the yeast powder (regarded as week 0) and 8 weeks after taking the capsule containing the yeast powder (regarded as week 8), the eight subjects were subjected to blood collection and analysis, skin detection, brain age detection and somatosensory questionnaire survey. The detection items of blood include the expression of anti-aging related genes in blood, the inflammatory index (content of CRP) in blood, and the content of LDL-C and the ratio of LDL-C to HDL-C in blood. The detection items of skin include skin wrinkle analysis and skin texture (roughness) analysis. The somatosensory questionnaire survey includes skin condition, hair loss condition and fatigue.

It should be noted that the statistically significant differences between the measurement results at week 0 and week 8 were statistically analyzed by the student's t-test.

### Example 8-1: Human body test-Blood analysis

Before the eight subjects take the yeast powder (at week 0) and after the eight subjects take the yeast powder (at week 8), purple head blood collection vessels containing an EDTA anticoagulant were respectively used for collecting 6 mL of venous blood from each subject, and the analysis of the expression of anti-aging related genes in blood, the analysis of the inflammatory index in blood, and the analysis of the content of LDL-C and the ratio of LDL-C to HDL-C in blood were conducted.

### Example 8-1-1: Analysis of expression of anti-aging related genes in blood

Herein, the detected anti-aging related genes include an SIRT1 gene (Gene ID: 23411) and an SOD3 gene (Gene ID: 6649).

First, the extracted venous blood was centrifuged at the rotating speed of 300 xg for 15 min. 2 mL of the leukocytic cream of the buffy coat was taken from the centrifuged venous blood, the leukocytic cream was diluted with 2 mL phosphate buffer (1X PBS, hereinafter referred to as 1X PBS buffer) to 4 mL, and then, the diluted leukocytic cream was slowly added to a centrifuge tube containing 3 mL of Ficoll-Paque Plus, where during addition, the diluted leukocytic cream and the Ficoll-Paque Plus need to be layered and cannot be mixed. Then, the centrifuge tube containing the layered diluted leukocytic cream and Ficoll-Paque Plus was centrifuged at 400 xg for 40 min, the supernatant was removed after centrifugation, and 2-3 mL of peripheral blood mononuclear cells (PBMC) in the middle layer in the centrifuge tube after centrifugation were taken. PBMC was rinsed with 3-times volume of the 1X PBS buffer and then mixed with the above 1X PBS buffer uniformly to form a PBMC mixture. Then, the PBMC mixture was centrifuged at the rotating speed of 300 xg for 10 min to form a PBMC supernatant and PBMC precipitates, the PBMC precipitates were taken and lysed with 600 µL of RNA lysis buffer, and then, RNA was extracted. Then, as described in the steps of Example 7, after the extracted RNA was reversely transcribed into cDNA, two groups of primers (SEQ ID NO: 19 and SEQ ID NO: 20, SEQ ID NO: 25 and SEQ ID NO: 26) in Table 2 were used for performing the quantitative real-time reverse transcription polymerase chain reaction on the cDNA respectively, so as to observe the expression of the SIRT1 gene and the SOD3 gene in blood at week 0 and week 8, as shown in FIG. 11.

**Table 2**

| Target gene | Primer name | Sequence number | Sequence | Primer length |
|---|---|---|---|---|
| SIRT1 | SIRT1-F | SEQ ID NO: 19 | TGCTGGCCTAATAGAGTGGCA | 21 |
| | SIRT1-R | SEQ ID NO:20 | CTCAGCGCCATGGAAAATGT | 20 |
| SOD3 | SOD3-F | SEQ ID NO:25 | AGCTGGAAAGGTGCCCGA | 18 |
| | SOD3-R | SEQ ID NO:26 | CTTGGCGTACATGTCTCGGAT | 23 |

In Table 2, F represents forward primer, and R represents reverse primer.

It should be noted that the gene expression in FIG. 11 was shown in relative expression percentage, where the STDEV formula of Excel software was used for computing the standard deviation, and the statistically significant differences between groups were statistically analyzed by the student's t-test. In FIG. 11, "*" represents that the p value was less than 0.05 compared with the control group.

Referring to FIG. 11, when the relative gene expression percentage of the SIRT1 gene and the *SOD3* gene of the eight subjects at week 0 was regarded as 100%, the relative gene expression percentage of the *SIRT1* gene at week 8 was 162.6%, and the relative gene expression percentage of the *SOD3* gene was 259.0%. It can be seen that when the subjects take the capsule containing 100 mg of the yeast powder daily for 8 consecutive weeks, the expression of the *SIRT1* gene and the SOD3 gene in the blood of the subjects was increased, indicating that the expression of the protein encoded by the *SIRT1* gene and the *SOD3* gene was increased. Moreover, as the downstream genes of NAD⁺, the *SIRT1* gene and the *SOD3* gene were related to the activity of mitochondria. When the SIRT1 gene and the *SOD3* gene in blood increase, it indicates that the content of NAD⁺ in blood increases, which activate mitochondria, thereby achieving the antiaging function. In other words, the expressions of the *SIRT1* gene and the *SOD3* gene in blood are increased by taking the yeast powder, indicating that the yeast powder has the potentials of activating mitochondria and resisting aging.

### Example 8-1-2: Analysis of inflammatory degree in blood

Herein, blood samples of the eight subjects were taken at week 0 and week 8 in this experiment, so as to detect the change of the inflammatory degree before and after the eight subjects take the capsule containing 100 mg of the yeast powder prepared in Example 1. CRP was a special protein produced by the liver, an acute phase reactant protein, and a pointer for tissue damage in an acute inflammatory reaction process. The enzyme-linked immunosorbent assay (ELISA) was used for measuring. The higher measured content of CRP in blood indicates more serious inflammatory reaction in the body. The content of CRP in the blood of the subjects in this embodiment was measured by Lezen Laboratory (Taiwan, China).

For the content of CRP, the STDEV formula of Excel software was used for computing the standard deviation, the statistically significant differences between groups were statistically analyzed by the student's t-test, and "*" represents that the p value was less than 0.05 compared with the control group.

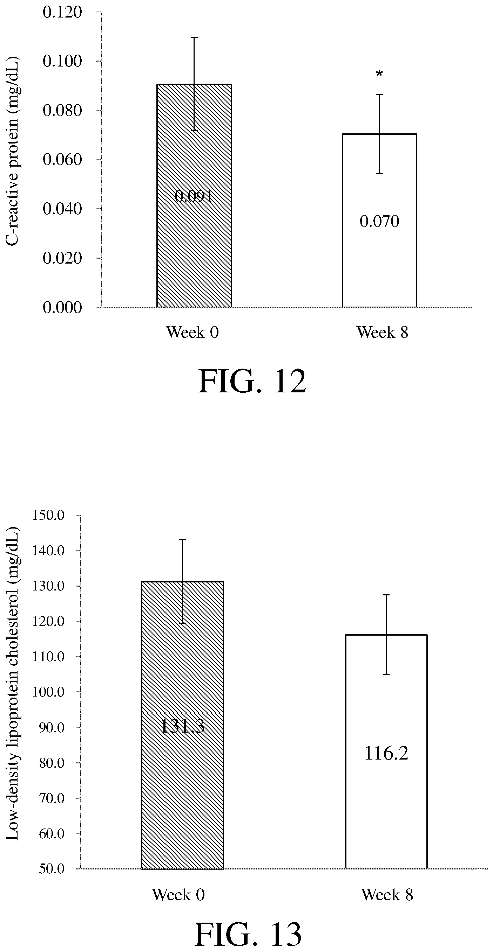

Referring to FIG. 12, the average content of CRP in the blood of the eight subjects at week 0 was 0.091 mg/dL, and the average content of CRP in the blood at week 8 was 0.070 mg/dL, reduced by 0.021 mg/dL (23.1%). In other words, the content of CRP in blood is reduced by taking the yeast powder, indicating that the yeast powder has the potential of resisting inflammation.

Therefore, after the subject takes 100 mg of the yeast powder daily, the inflammatory index (content of CRP) is significantly reduced, thereby reducing uncomfortable symptoms of chronic inflammation.

### Example 8-1-2: Analysis of blood fat in blood

LDL-C will invade endothelial cells and cause cells to be oxidized, thus increasing the risk of cardiovascular diseases. Therefore, it is generally expected to reduce the content of LDL-C. On the contrary, HDL-C can inhibit cardiovascular diseases, and any cholesterol in HDL particles can be regarded as a factor to protect the health of the cardiovascular system of the body. Therefore, it is generally expected to increase the content of HDL-C.

Herein, the analysis of blood fat includes detection of the content of LDL-C and the ratio of LDL-C to HDL-C, measured by Lezen Laboratory (Taiwan, China).

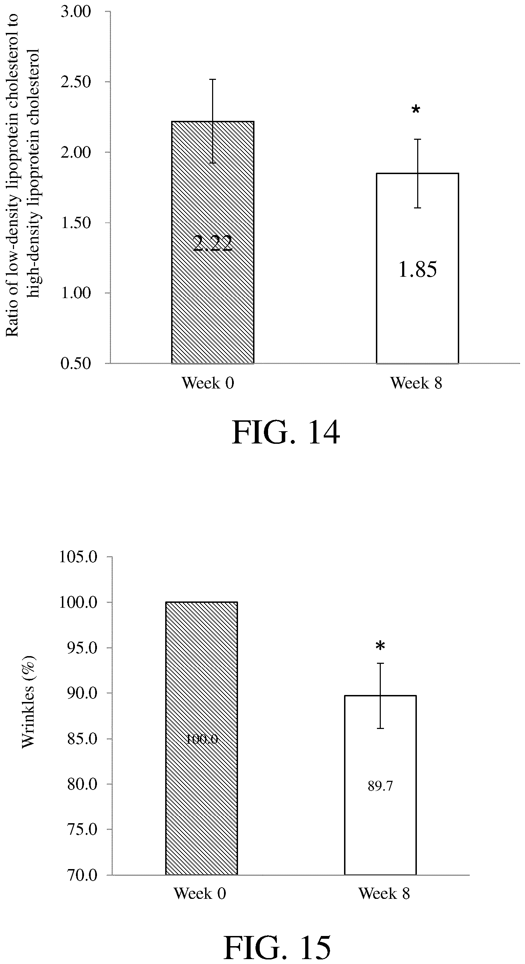

For the blood collected from the eight subjects at week 0 and week 8, the content of LDL-C and the ratio of LDL-C to HDL-C were detected respectively, as shown in FIG. 13 and FIG. 14. Moreover, for the content of LDL-C in FIG. 13 and the ratio of LDL-C to HDL-C in FIG. 14, the STDEV formula of Excel software was used for computing the standard deviation, the statistically significant differences between groups were statistically analyzed by the student's t-test, and "*" represents that the p value was less than 0.05 compared with the control group.

Referring to FIG. 13, the average content of LDL-C in the blood of the eight subjects at week 0 was 131.3 mg/dL, and the average content of LDL-C in the blood at week 8 was 116.2 mg/dL. It can be seen that after the eight subjects take the yeast powder for 8 weeks, the content of LDL-C in the blood was reduced by 15.1 mg/dL, indicating that the average content of LDL-C in the blood of the eight subjects at week 8 was reduced by 11.5% compared with the average content of LDL-C in the blood of the eight subjects at week 0. In other words, the content of LDL-C in blood was reduced by taking the yeast powder so as to prevent arteriosclerosis caused by the oxidation of LDL, indicating that the yeast powder has the potentials of regulating blood fat and achieving cardiovascular health care.

Referring to FIG. 14, the average ratio of LDL-C to HDL-C in the blood of the eight subjects at week 0 was 2.22, and the average ratio of LDL-C to HDL-C in the blood at week 8 was 1.85, reduced by 0.37 (16.7%). Since higher ratio of LDL-C to HDL-C represents a higher risk of coronary arteriosclerosis, reducing the ratio of LDL-C to HDL-C represents a reduced risk of coronary arteriosclerosis. In other words, the ratio of LDL-C to HDL-C in blood is regulated and reduced by taking the yeast powder, indicating that the yeast powder has the potentials of regulating blood fat, reducing the risk of coronary arteriosclerosis, and achieving cardiovascular health care.

Therefore, after the subject takes 100 mg of the yeast powder daily, the inflammatory index (content of CRP) is significantly reduced, and the risk of coronary arteriosclerosis is reduced, thereby reducing the risk of cardiovascular diseases and achieving the function of cardiovascular health care.

### Example 8-2: Human body test-Skin analysis

Herein, a VISIA Complexion Analysis System (Canfield scientific, USA) was used for analyzing skin conditions. For skin wrinkles, the facial skin of the same subj ect before and after taking the yeast powder was photographed with a high-resolution camera lens, the change of the skin shadow can be detected by the irradiation of standard white light, then texture positions can be detected, and a value can be obtained to indicate the smoothness of the skin. For skin textures, the facial skin of the same subject before and after taking the yeast powder was detected through the principle of photographing high-resolution skin images with visible light and analyzing roughness with build-in software according to the concave and convex skin. A higher measured value represents rougher skin.

Referring to FIG. 15, the average skin wrinkle percentage detected by the VISIA Complexion Analysis System before the eight subjects took the yeast powder (at week 0) was regarded as 100%. After the subjects took the yeast powder for 8 consecutive weeks, the average skin wrinkle percentage was reduced to 89.7%. In other words, compared with before taking the yeast powder (at week 0), after the subjects took the capsule containing 100 mg of the yeast powder for 8 consecutive weeks, the skin wrinkle percentage of the subjects were reduced by 10.3%. It can be seen that by taking the yeast powder rich in NMN for a long term, the skin wrinkles of the subject is reduced, and the skin conditions of the subject is improved, that is, the yeast powder has the effect of smoothing fine wrinkles.

Moreover, before one of the subjects takes the yeast powder (at week 0), more and dense wrinkles WK-0 were photographed by the VISIA Complexion Analysis System; and after the subject takes the yeast powder for 8 weeks (at week 8), the number of the photographed wrinkles WK-8 was reduced, and the photographed wrinkles WK-8 were relatively sparse. Therefore, by taking the yeast powder rich in NMN for a long term, the number of wrinkles on the face of the subject is reduced.

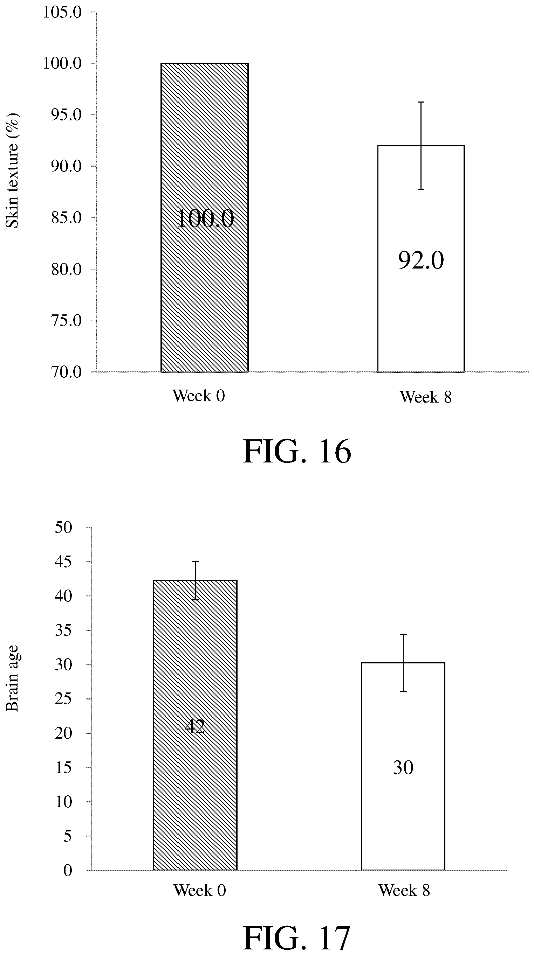

Referring to FIG. 16, the average skin texture percentage detected by the VISIA Complexion Analysis System before the eight subjects take the yeast powder (at week 0) was regarded as 100%. After the subjects take the yeast powder for 8 consecutive weeks, the average skin texture percentage was reduced to 92.0%. In other words, compared with before taking the yeast powder (at week 0), after the subjects took the capsule containing 100 mg of the yeast powder for 8 consecutive weeks, the skin texture percentage of the subjects can be reduced by 8.0%. It can be seen that by taking the yeast powder rich in NMN for a long term, the skin textures of the subject can be reduced, and the skin roughness of the subject can be reduced, so as to achieve the effect of improving the skin conditions of the subject, that is, the yeast powder has the effect of making the skin delicate.

Moreover, before one of the subjects takes the yeast powder (at week 0), more and dense concave and convex positions T-0 were photographed by the VISIA Complexion Analysis System; and after the subject takes the yeast powder for 8 weeks (at week 8), the number of the photographed concave and convex positions T-8 was reduced, and the photographed concave and convex positions T-8 were relatively sparse. Therefore, by taking the yeast powder rich in NMN for a long term, the concave and convex positions on the face of the subject are reduced, thereby reducing the roughness of the skin and making the skin of the subject delicate.

### Example 8-3: Human body test-Brain age analysis

Herein, before the eight subjects take the yeast powder (at week 0) and after the eight subjects take the yeast powder (at week 8), the "Test Your Brain Age" software was used for testing the brain age of the eight subjects. Specifically, the "Test Your Brain Age" software computes the brain age of the subject according to the overall correct answer rate and response speed of the subject, and the test item includes selection of the numbers displayed on a screen from small to large before the numbers displayed on the screen disappear.

Website of the "Test Your Brain Age" software: https://apps.apple.com/tw/app/test-your-brain-age/id969455998.

Referring to FIG. 17, the average brain age of the eight subjects before taking the yeast powder (at week 0) was 42 years old. After the subjects take the yeast powder for 8 consecutive weeks, the average brain age of the subjects was reduced to 30 years old. In other words, compared with before taking the yeast powder (at week 0), after the subjects take the capsule containing 100 mg of the yeast powder for 8 consecutive weeks, the average brain age of the subjects was reduced by 12 years old, indicating that after the subjects take the yeast powder for 8 weeks, the brain was clearer, and the response speed to answering questions was increased.

It can be seen that by taking the yeast powder rich in NMN for a long term, the brain can be clearer, and the brain detection age of the subject can be reduced, thereby achieving the effect of resisting aging, that is, the yeast powder has the effects of making the brain clear and resisting aging.

### Example 8-4: Human body test-Somatosensory questionnaire survey

Herein, the subjects fill in the somatosensory questionnaire at week 0 and week 8 respectively, and the somatosensory questionnaire includes the comprehensive evaluation of physical conditions. The severity was evaluated according to the physical conditions (such as poor overall skin condition, hair loss condition, hair thinning condition and physical fatigue) of the subjects, as shown in Table 3.

**Table 3**

| Comprehensive evaluation of physical conditions | | | | | |
|---|---|---|---|---|---|
| Symptoms | Severity | | | | |
| | None (1) | Mild (2) | Obvious (3) | Serious (4) | Very serious (5) |
| 1. Poor overall skin condition | | | | | |
| 2. Hair loss condition | | | | | |
| 3. Hair thinning condition | | | | | |
| 4. Physical fatigue | | | | | |

In Table 3, "none" represents 1 score, "mild" represents 2 scores, "obvious" represents 3 scores, "serious" represents 4 scores, and "very serious" represents 5 scores. The severity of each test item was counted by summing up the scores.

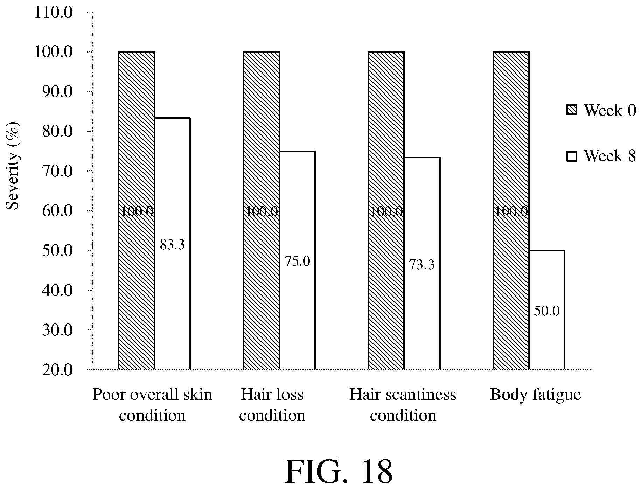

Referring to FIG. 18, the average severity of the "poor overall skin condition" of the eight subjects before taking the yeast powder (at week 0) was regarded as 100%. After the eight subjects take the yeast powder for 8 consecutive weeks (at week 8), the average severity of the "poor overall skin condition" was reduced to 83.3%. In other words, compared with before taking the yeast powder (at week 0), after the eight subjects took the capsule containing the yeast powder for 8 consecutive weeks, the feeling of the eight subjects about the "poor overall skin condition" were reduced by 16.7%, indicating that the yeast powder rich in NMN improved the skin conditions of the subj ect.

The average severity of the "hair loss condition" of the eight subjects before taking the yeast powder (at week 0) was regarded as 100%. After the eight subjects take the yeast powder for 8 consecutive weeks (at week 8), the average severity of the "hair loss condition" was reduced to 75.0%. In other words, compared with before taking the yeast powder (at week 0), after the eight subjects took the capsule containing the yeast powder for 8 consecutive weeks, the feeling of the eight subjects about the "hair loss condition" were reduced by 25.0%, indicating that the yeast powder rich in NMN reduced the hair loss condition of the subject.

The average severity of the "hair thinning condition" of the eight subjects before taking the yeast powder (at week 0) was regarded as 100%. After the eight subjects take the yeast powder for 8 consecutive weeks (at week 8), the average severity of the "hair thinning condition" was reduced to 73.3%. In other words, compared with before taking the yeast powder (at week 0), after the eight subjects took the capsule containing the yeast powder for 8 consecutive weeks, the feeling of the eight subjects about the "hair thinning condition" were reduced by 26.7%, indicating that the yeast powder rich in NMN reduced the hair thinning condition of the subject and increase the hair density of the subj ect.

The average severity of the "physical fatigue" of the eight subjects before taking the yeast powder (at week 0) was regarded as 100%. After the eight subjects take the yeast powder for 8 consecutive weeks (at week 8), the average severity of the "physical fatigue" was reduced to 50.0%. In other words, compared with before taking the yeast powder (at week 0), after the eight subjects took the capsule containing the yeast powder for 8 consecutive weeks, the feeling of the eight subjects about the "physical fatigue" were reduced by 50.0%, indicating that the yeast powder rich in NMN reduces the physical fatigue of the subject, improve sthe spirit of the subject, and made the subject more energetic.

It can be seen that by taking the yeast powder rich in NMN for a long term, the overall skin condition, hair loss condition and fatigue of the subject can be improved.

### Example 9: Sequencing analysis of peptide in yeast powder rich in NMN

The yeast powder rich in NMN, prepared in Example 1, was appropriately diluted and then centrifuged at 13000 rpm for 2 min, 200 µL of supernatant was sucked, a C18-ZipTip (Millpore) was used for desalting and concentrating, after the sample was redissolved, 1/2 volume of the sample was taken, and LC-MS/MS analysis was performed under the following conditions. An MS/MS spectrum uses a Mascot analysis program to search and analyze the database to obtain analysis results, thereby obtaining the protein sequence and identification information of the peptide contained in the above yeast powder.

### Reaction conditions:

Mass spectrometer: LTQ XL (Thermo Scientific)
LC system: Agilent 1200 Series
Buffer solution A: ddH₂O / 0.1% formic acid
Buffer solution B: 100% ACN / 0.1% formic acid
Analytical tube column: C18 reverse phase column
Gradients: B% was 5% at 0.00 min, B% was 5% at 10.02 min, B% was 5% at 10.05 min, B% was 40% at 45.00 min, B% was 85% at 50.00 min, B% was 85% at 60.00 min, B% was 5% at 63.00 min, B% was 5% at 75.02 min, B% was 5% at 75.05 min, and B% was 5% at 90.00 min.
Analysis results: the protein identification information of the peptide contained in the above yeast powder rich in NMN was Q₁₀ biosynthetic protein, and the protein sequence of the peptide analyzed was MVAKAHSKK (SEQ ID NO: 27). It can be seen that the yeast powder rich in NMN contains peptide fragments of the Q₁₀ biosynthetic protein.

In conclusion, the yeast powder which contains at least 5000 ppm of NMN can be prepared according to the preparation method of the yeast powder rich in NMN in any embodiment of the present invention. The present invention solves the problem that the traditional NMN can only be chemically synthesized and thus harmful by-products are generated in the preparation process, and also solves the technical bottleneck that the traditional NMN is inedible and can only be used externally. The components of the fermentation medium used in the preparation method of any embodiment include NAM, tryptophan and niacin. Moreover, the components of the fermentation medium used in the preparation method of any embodiment further include any one or more of Solanum plant extracts, Aronia plant extracts and Musa plant extracts. The yeast powder prepared by the preparation method of any embodiment can be used for preparing a composition for improvement of skin conditions, hair care, anti-inflammation, cardiovascular health care, antioxidation, antiaging, and/or improvement of body fatigue. In other words, the above composition has one or more of the following functions of resisting cell oxidation pressure, increasing the activity of mitochondria in cells, regulating anti-aging related genes (that is, inhibiting the *PARP2* gene and improving the *CCT* genes, the *Parkin* gene, the *Atg1* gene, the *Atg8* genes, the *FOXO* gene, the *SIRT1* gene, the *NADSYN* gene, the *MRPS5* gene and the *SOD3* gene), reducing skin wrinkles and skin textures, making the brain younger (reducing brain age), reducing the hair loss condition, reducing the degree of hair scantiness, and reducing the physical fatigue. Moreover, the yeast powder of any embodiment further includes Q₁₀ biosynthetic protein.

Although the technical contents of the present invention have been disclosed above with preferred embodiments, but are not intended to limit the present invention. Some changes and modifications made by those skilled in the art without departing from the spirit of the present invention shall be covered in the scope of the present invention. Therefore, the protection scope of the present invention shall be defined by the scope of the appended claims.

Of course, many other embodiments of the present invention may be provided. Without departing from the spirit and essence of the present invention, those skilled in the art can make various corresponding changes and deformations according to the present invention, but these corresponding changes and deformations shall belong to the protection scope of the claims of the present invention.

### [Deposit of biological materials]

Food Industry Research and Development Institute (Taiwan, China); October 24, 2019; Deposit No.: BCRC 920118.

German Collection of Microorganisms and Cell Cultures (Germany); March 27, 2020; Deposit No.: DSM33480.

### Industrial applicability

The present invention discloses a yeast powder rich in NMN, and a preparation method and applications thereof. The preparation method includes: a first medium, a second medium and a third medium are prepared; a yeast is inoculated into the first medium for fermentation to obtain a first fermentation broth; the first fermentation broth is taken and inoculated into the second medium for fermentation to obtain a second fermentation broth; the second fermentation broth is taken and inoculated into the third medium for fermentation to obtain a third fermentation broth; and the third fermentation broth is centrifuged to obtain a fermented product, and the fermented product is dried to obtain the yeast powder. The components of the first medium, the second medium and the third medium include NAM, tryptophan and niacin. The content of NMN in the yeast powder is at least 5000 ppm.

## Claims

1. A preparation method of a yeast powder rich in nicotinamide mononucleotide (NMN), comprising:
preparing a first medium, a second medium and a third medium, wherein components of the first medium, the second medium and the third medium comprise nicotinamide (NAM), tryptophan and niacin;
inoculating the first medium with yeast for fermentation to obtain a first fermentation broth;
inoculating the second medium with the first fermentation broth for fermentation to obtain a second fermentation broth;
inoculating the third medium with the second fermentation broth for fermentation to obtain a third fermentation broth; and
centrifuging the third fermentation broth to obtain a fermented product, and drying the fermented product to obtain the yeast powder, wherein the content of the NMN in the yeast powder is at least 5000 ppm.

2. The preparation method according to claim 1, wherein the first medium is inoculated with the yeastat an inoculation amount of 5 vol%; the first second medium is inoculated with the first fermentation broth at an inoculation amount of 6 vol%; and the third medium is inoculated with the second fermentation broth at an inoculation amount of 10 vol%.

3. The preparation method according to claim 1, wherein a concentration of the NAM is 0.01-0.3 wt%, a concentration of the tryptophan is 0.1-0.5 wt%, and a concentration of the niacin is 0.001-0.06 wt%.

4. The preparation method according to claim 1, wherein the volume ratio of the first medium to the second medium to the third medium is 3:50:500.

5. The preparation method according to claim 1, wherein the yeast powder further comprises Q₁₀ biosynthetic protein.

6. The preparation method according to claim 1, wherein the components of the first medium, the second medium and the third medium further comprise any one or more of *Solanum* plant extracts, *Aronia* plant extracts and *Musa* plant extracts.

7. A yeast powder, prepared by the preparation method according to claim 1, wherein the content of NMN in the yeast powder is at least 5000 ppm.

8. The yeast powder according to claim 7, wherein the yeast powder further comprises Q₁₀ biosynthetic protein.

9. An application of a yeast powder for preparing a composition for improvement of skin conditions, hair care, anti-inflammation, cardiovascular health care, antioxidation, antiaging, and/or improvement of body fatigue, wherein the yeast powder is prepared by the preparation method according to claim 1, and the content of NMN in the yeast powder is at least 5000 ppm.

10. The application according to claim 9, wherein the yeast powder further comprises Q₁₀ biosynthetic protein.

11. The application according to claim 9, wherein the improvement of skin conditions is to enhance skin tightness, reduce wrinkles, reduce skin roughness, or combinations thereof.

12. The application according to claim 9, wherein the hair care is to reduce hair loss or reduce the degree of hair scantiness.

13. The application according to claim 9, wherein the yeast powder is used to regulate blood fat.

14. The application according to claim 13, wherein the yeast powder is used to reduce the expression of C-reactive protein (CRP) of a subject.

15. The application according to claim 9, wherein the yeast powder achieves the antiaging function by regulating the expression of an anti-aging related gene, activating mitochondria or reducing brain age.

16. The application according to claim 15, wherein the anti-aging related genes comprise *CCT* genes, *PARP2* gene, *Parkin* gene, *Atg* genes, *FOXO* gene, *SIRT1* gene, *NADSYN* gene, *MRPS5* gene, *SOD3* gene or combinations thereof.

17. The application according to claim 9, wherein the yeast powder achieves the antioxidation function by reducing the damage to reactive oxygen species (ROS).

18. The application according to claim 9, wherein a daily dose of the yeast powder is 100 mg.
